# EUROPEAN PATENT APPLICATION

(11) **EP 3 643 276 A1**
(43) Date of publication of application: **29.04.2020**
(21) Application number: 18820566.0
(22) Date of filing: 21.06.2018
(51) Int. Cl.: A61F 5/56, F16B 39/24, F16B 39/32, F16B 35/06

(54) **MOUTHPIECE**

(30) Priority: 22.06.2017 JP 2017122364; 15.02.2018 JP 2018025034; 26.03.2018 JP 2018058743
(71) Applicant: Kurume University, Kurume-shi Fukuoka 830-0011 (JP); Mitsui Chemicals, Inc., Minato-ku Tokyo 105-7122 (JP)
(72) Inventor: KUSUKAWA, Jingo, Fukuoka 8300011 (JP); TANOUE, Ryuichiro, Fukuoka 8300011 (JP); OGATA, Kinuko, Fukuoka 8300011 (JP); YAZAKI, Kohei, Nagano 3910012 (JP); HAMA, Daigo, Nagano 3910012 (JP); OOWA, Junji, Nagano 3910012 (JP); YUKITA, Takashi, Chiba 2990265 (JP); NAGAI, Hideyuki, Yamaguchi 7400061 (JP); HASEGAWA, Akira, Chiba 2990265 (JP); TSUCHIYA, Yasufumi, Tokyo 1057122 (JP)
(74) Representative: Epping - Hermann - Fischer
(86) International application number: PCT/JP2018/023550
(87) International publication number: WO 2018/235888

(57) **Abstract**

A mouthpiece with which the advancement amount of a lower jaw mouthpiece can be more easily adjusted, without increasing the size of the mouthpiece, is provided. The mouthpiece has: a pair of upper jaw and lower jaw mouthpieces facing each other; a male screw; a female screw; a front holding part provided to one of the mouthpieces, holding the male screw or the female screw; and a rear holding part provided to the other mouthpiece, holding the female screw or the male screw further rearward than the front holding part. The female screw and the male screw position the lower jaw mouthpiece in the front-rear direction with respect to the upper jaw mouthpiece, as a result of the turning of the male screw which is screwed into the female screw at a denture tooth surface or further rearward than the denture tooth surface.

## Description

### Technical Field

The present invention relates to an oral appliance.

### Background Art

In recent years, an oral appliance is used for the prevention, treatment, and the like of sleep apnea syndrome.

The oral appliance can prevent respiratory arrest or hypopnea during sleep by preventing the constriction of the trachea by limiting the displacement of the lower jaw of a user wearing the oral appliance to the rear side by wearing the oral appliance such that a lower-jaw oral appliance is positioned frontward of an upper-jaw oral appliance. The oral appliance can cause the trachea of the user to be less likely to be constricted by adjusting the advance amount of the lower-jaw oral appliance with respect to the upper-jaw oral appliance and moving the lower jaw frontward of the upper jaw by an amount fitted for the shape of the jaw of the user.

For example, Patent Literature (hereinafter, abbreviated as PTL) 1 discloses a hook-shaped lower-jaw advancing apparatus that is fixed to a curved-shape member (upper-jaw oral appliance) on the upper jaw side and hooked on a curved-shape member (lower-jaw oral appliance) on the lower jaw side out of one pair of curved-shape members (the upper-jaw oral appliance and the lower-jaw oral appliance) facing each other, to thereby adjust the positions of the curved-shape members forward and backward. The lower-jaw advancing apparatus is positioned by a screw member, and sets the front and rear positions of the curved-shape members.

PTL 2 discloses a lower-jaw advancing apparatus capable of adjusting the positions of an upper-jaw oral appliance and a lower-jaw oral appliance in the front-rear direction by a male screw retained by the upper-jaw oral appliance and a female screw retained by the lower-jaw oral appliance. In the lower-jaw advancing apparatus disclosed in PTL 2, the male screw and the female screw can be disposed in the front portion of an oral appliance, and hence discomfort due to restriction of the movement of the tongue is conceived to be reduced unlike the hook-shaped fastener disclosed in PTL 1.

PTL 3 discloses a lower-jaw advancing apparatus capable of adjusting the position of an upper-jaw oral appliance and a lower-jaw oral appliance in the front-rear direction by one pair of tongue-like portions extending from both of the upper-jaw oral appliance and the lower-jaw oral appliance to the front side, and locking posts that pass through any of a plurality of locking holes formed in the tongue-like portions and positions the tongue-like portions with respect to each other. In the lower-jaw advancing apparatus disclosed in PTL 3, positioning of the upper-jaw oral appliance and the lower-jaw oral appliance is conceived to be easy because the advance amount of the lower-jaw oral appliance can be adjusted by selecting the locking holes through which the locking posts pass, as appropriate.

### Citation List

### Patent Literature

PTL 1
   U.S. Patent Application Publication No. 2002/0000230
PTL 2
   Japanese Unexamined Patent Application Publication (Translation of PCT Application) No. 2007-501641
PTL 3
   Japanese Unexamined Patent Application Publication (Translation of PCT Application) No. 2013-523221

### Summary of Invention

### Technical Problem

The lower-jaw advancing apparatus disclosed in PTL 1 adjusts the position of the lower-jaw advancing apparatus with respect to the upper-jaw oral appliance by the rotation of the screws. Then, the lower-jaw advancing apparatus is fixed to the upper-jaw oral appliance, and is hooked on the lower-jaw oral appliance. Therefore, when the lower-jaw advancing apparatus is positioned, it is difficult to intuitively recognize the advance amount of the lower-jaw oral appliance, and the position adjustment of the lower-jaw advancing apparatus for advancing the lower-jaw oral appliance tends to be time-consuming.

Meanwhile, the lower-jaw advancing apparatuses disclosed in PTL 2 and PTL 3 can adjust the advance amount of the lower-jaw oral appliance in a state in which the upper-jaw oral appliance and the lower-jaw oral appliance are disposed so as to face each other, and hence the position adjustment of the lower-jaw advancing apparatus for advancing the lower-jaw oral appliance is easier. However, the lower-jaw advancing apparatuses disclosed in PTL 2 and PTL 3 are disposed frontward of the dentition. Therefore, the wearing comfortability of the user may decrease because the oral appliance needs to be upsized, the lower-jaw advancing apparatus may abut against the lips, and the closing of the mouth by closing the lips together becomes difficult by the lower-jaw advancing apparatus, for example.

In view of the problems described above, an object of the present invention is to provide an oral appliance capable of adjusting the advance amount of a lower-jaw oral appliance in an easier manner without upsizing the oral appliance in a first aspect thereof.

In the lower-jaw advancing apparatuses disclosed in PTL 1 and PTL 2, when the screws rotate by its own by vibration and the like when the oral appliance is used, the positions of the upper-jaw oral appliance and the lower-jaw oral appliance may be out of the optimal positions.

Meanwhile, in the lower-jaw advancing apparatus disclosed in PTL 3, the advance amount of the lower-jaw oral appliance can be set in accordance with the combination of the locking holes, and hence the positional misalignment of the upper-jaw oral appliance and the lower-jaw oral appliance hardly occurs. However, the lower-jaw advancing apparatus disclosed in PTL 3 needs at least two locking posts in order to position the lower-jaw oral appliance, and also needs locking members for retaining those locking posts. Therefore, the oral appliance including the lower-jaw advancing apparatus may be upsized, and the wearing comfortability of the user may decrease.

In view of the problems described above, an object of the present invention is to provide an oral appliance capable of suppressing the positional misalignment of an upper-jaw oral appliance and a lower-jaw oral appliance without upsizing the oral appliance in a second aspect thereof.

### Solution to Problem

An oral appliance according to one mode of a first aspect of the present invention for solving the problems mentioned above includes: one pair of an upper-jaw oral appliance and a lower-jaw oral appliance facing each other; a male screw; a female screw; a front-side retaining portion that is disposed on one oral appliance out of the upper-jaw oral appliance and the lower-jaw oral appliance and retains the male screw or the female screw; and a rear-side retaining portion that is disposed on another oral appliance out of the upper-jaw oral appliance and the lower-jaw oral appliance and retains the female screw or the male screw behind the front-side retaining portion, in which the front-side disposing portion is disposed on a dentition surface of the one oral appliance or behind the dentition surface, and the female screw and the male screw serve to position the lower-jaw oral appliance with respect to the upper-jaw oral appliance in a front-rear direction by rotation of the male screw threaded into the female screw on the dentition surface or behind the dentition surface.

An oral appliance according to one mode of a second aspect of the present invention for solving the problems mentioned above includes: an upper-jaw oral appliance and a lower-jaw oral appliance; a female screw; a male screw; a fixed member that is fixed to the upper-jaw oral appliance or the lower-jaw oral appliance and retains the female screw; and a sliding member that is fixed to the lower-jaw oral appliance or the upper-jaw oral appliance and retains the male screw, in which the male screw and the female screw serve to position the sliding member with respect to the fixed member in the front-rear direction by the rotation of the male screw threaded into the female screw, and position the lower-jaw oral appliance with respect to the upper-jaw oral appliance in the front-rear direction. The oral appliance further includes a stopper that regulates rotation of the male screw with respect to the female screw with respect to the axial direction.

### Advantageous Effects of Invention

According to the first aspect of the present invention, the oral appliance capable of adjusting the advance amount of the lower-jaw oral appliance in an easier manner without upsizing the oral appliance is provided.

According to the second aspect of the present invention, the oral appliance capable of suppressing the positional misalignment of the upper-jaw oral appliance and the lower-jaw oral appliance without upsizing the oral appliance is provided.

### Brief Description of Drawings

FIG 1 is a schematic perspective view illustrating the configuration of an oral appliance according to Embodiment 1 of the present invention;
FIG 2 is a side view illustrating how the lower jaw is advanced while the oral appliance according to Embodiment 1 of the present invention is being worn;
FIG 3 is a schematic perspective view of a lower-jaw advancing apparatus according to Embodiment 1 of the present invention;
FIG 4 is a schematic exploded perspective view of the lower-jaw advancing apparatus according to Embodiment 1 of the present invention;
FIG 5A is a perspective view schematically illustrating a first configuration of a front-side retaining portion in Embodiment 2 of the present invention, and FIG. 5B is a cross-sectional plan view schematically illustrating the first configuration described above;
FIG. 6A is a front view schematically illustrating a second configuration of the front-side retaining portion in Embodiment 2 of the present invention, FIG. 6B is a cross-sectional view schematically illustrating the second configuration described above, and FIG. 6C is a partial perspective view schematically illustrating the second configuration described above;
FIG. 7A is a front view schematically illustrating a third configuration of the front-side retaining portion in Embodiment 2 of the present invention, FIG. 7B is a cross-sectional view schematically illustrating the third configuration described above, and FIG. 7C is a partial perspective view schematically illustrating the third configuration described above;
FIG. 8A is a front view schematically illustrating a fourth configuration of the front-side retaining portion in Embodiment 2 of the present invention, FIG. 8B is a cross-sectional view schematically illustrating the fourth configuration described above, and FIG. 8C is a partial perspective view schematically illustrating the fourth configuration described above;
FIG. 9A is a front view schematically illustrating a first configuration of a rear-side retaining portion in Embodiment 2 of the present invention, FIG. 9B is a cross-sectional view schematically illustrating the first configuration described above, and FIG. 9C is a partial perspective view schematically illustrating the first configuration described above;
FIG. 10A is a front view schematically illustrating a second configuration of the rear-side retaining portion in Embodiment 2 of the present invention, FIG. 10B is a cross-sectional view schematically illustrating the second configuration described above, and FIG. 10C is a partial perspective view schematically illustrating the second configuration described above;
FIG. 11A is a perspective view schematically illustrating a third configuration of the rear-side retaining portion in Embodiment 2 of the present invention, and FIG. 11B is a cross-sectional plan view schematically illustrating the third configuration described above;
FIG. 12A is a perspective view schematically illustrating a fourth configuration of the rear-side retaining portion in Embodiment 2 of the present invention, and FIG. 12B is a cross-sectional plan view schematically illustrating the fourth configuration described above;
FIG. 13 is a side view schematically illustrating the configuration of an oral appliance according to Embodiment 3 of the present invention;
FIG. 14 is a schematic perspective view illustrating the configuration of the oral appliance according to the present invention;
FIG. 15 is a side view illustrating how the lower jaw is advanced while the oral appliance according to the present invention is being worn;
FIG. 16 is a schematic perspective view of a lower-jaw advancing apparatus according to Embodiment 4 of the present invention;
FIG. 17 is a schematic exploded perspective view of the lower-jaw advancing apparatus according to Embodiment 4 of the present invention;
FIG. 18 is a transparent view of a distal end portion of the lower-jaw advancing apparatus according to Embodiment 4 of the present invention;
FIG. 19 is a perspective view of a distal end portion of a lower-jaw advancing apparatus according to Embodiment 5 of the present invention;
FIG. 20 is a cross-sectional view of the lower-jaw advancing apparatus according to Embodiment 5 of the present invention taken along line A-A in FIG. 19;
FIG. 21 is a schematic perspective view of a lower-jaw advancing apparatus according to Embodiment 6 of the present invention;
FIG. 22 is a schematic exploded perspective view of the lower-jaw advancing apparatus according to Embodiment 6 of the present invention;
FIG. 23 is a schematic exploded perspective view of a lower-jaw advancing apparatus according to Embodiment 7 of the present invention;
FIG. 24 is a schematic exploded perspective view of a lower-jaw advancing apparatus according to Embodiment 8 of the present invention; and
FIG. 25 is a schematic enlarged perspective view of a distal end portion of the lower-jaw advancing apparatus according to Embodiment 8 of the present invention.

### Description of Embodiments

An oral appliance of the present invention is described below with use of a plurality of embodiments.

Note that, in the description below, the "front side" and the "rear side" mean the direction toward the front side of a user wearing the oral appliance (the direction toward the lips side when seen from the body of the tongue in the oral cavity) and the direction toward the rear side thereof (the direction toward the throat side when seen from the body of the tongue in the oral cavity), the "left-right direction" means the left direction and the right direction with respect to the medial line of the upper jaw of the user wearing the oral appliance when facing toward the front side of the user (specifically, the direction toward the cheeks on both sides when seen from the medial line of the upper jaw), the "mouth-opening direction" means the direction in which the lower jaw moves when the user wearing the oral appliance opens the mouth, the "dentition surface" means a surface equivalent to the front surface of the teeth of the user wearing the oral appliance, and the "inner periphery" and the "outer periphery" mean the peripheral surface on the side close to the body surface of the user wearing the oral appliance and the peripheral surface on the side far from the body surface.

### 1. Oral Appliance according to First Aspect

### [Embodiment 1]

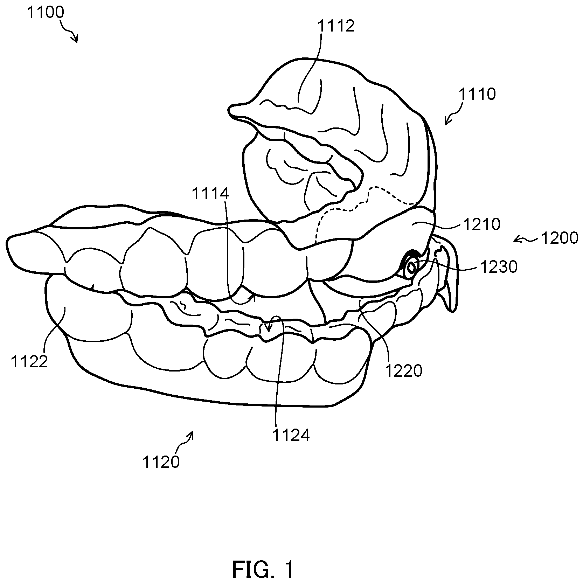

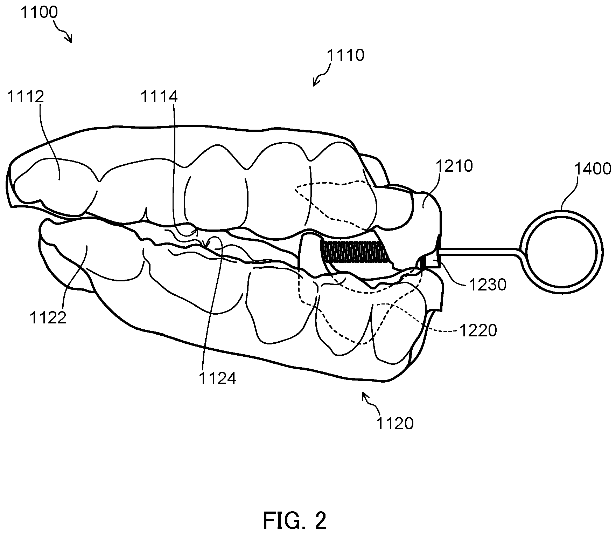

Oral appliance 1100 according to Embodiment 1 of the present invention includes one pair of an upper-jaw oral appliance and a lower-jaw oral appliance facing each other and lower-jaw advancing apparatus 1200 as illustrated in FIG. 1 that is a schematic perspective view illustrating the configuration of oral appliance 1100, and FIG. 2 that is a side view illustrating how the lower jaw is advanced while the oral appliance is being worn.

Upper-jaw oral appliance 1110 has dentition impression portion 1112 that can be worn on the dentition of the upper jaw of the user formed therein, and is formed so as to be wearable on the upper jaw of the user. Lower-jaw oral appliance 1120 has dentition impression portion 1122 that can be worn on the dentition of the lower jaw of the user formed therein, and is formed so as to be wearable on the lower jaw of the user. Lower-jaw oral appliance 1120 can be coupled to upper-jaw oral appliance 1110 disposed so as to face lower-jaw oral appliance 1120, and can be positioned frontward of upper-jaw oral appliance 1110 by lower-jaw advancing apparatus 1200.

Note that the region in upper-jaw oral appliance 1110 that faces lower-jaw oral appliance 1120 when the user wearing oral appliance 1100 is closing the mouth (hereinafter also simply referred to as "when the mouth is closed") is hereinafter referred to as downward facing surface 1114, and the region in lower-jaw oral appliance 1120 that faces upper-jaw oral appliance 1110 when the mouth is closed is hereinafter referred to as upward facing surface 1124. The surfaces on upper-jaw oral appliance 1110 and lower-jaw oral appliance 1120 that face the tongue portion side of the user wearing the oral appliance are referred to as tongue-side surfaces, and the surfaces thereof facing the lips side are referred to as lip-side surfaces.

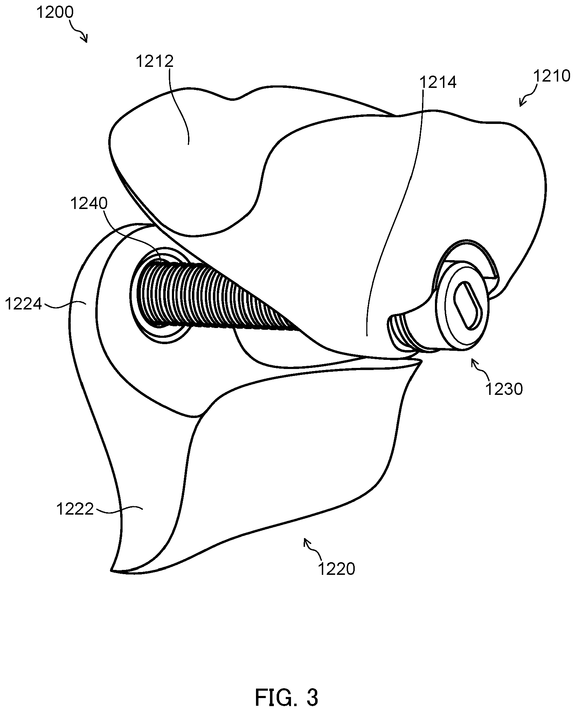

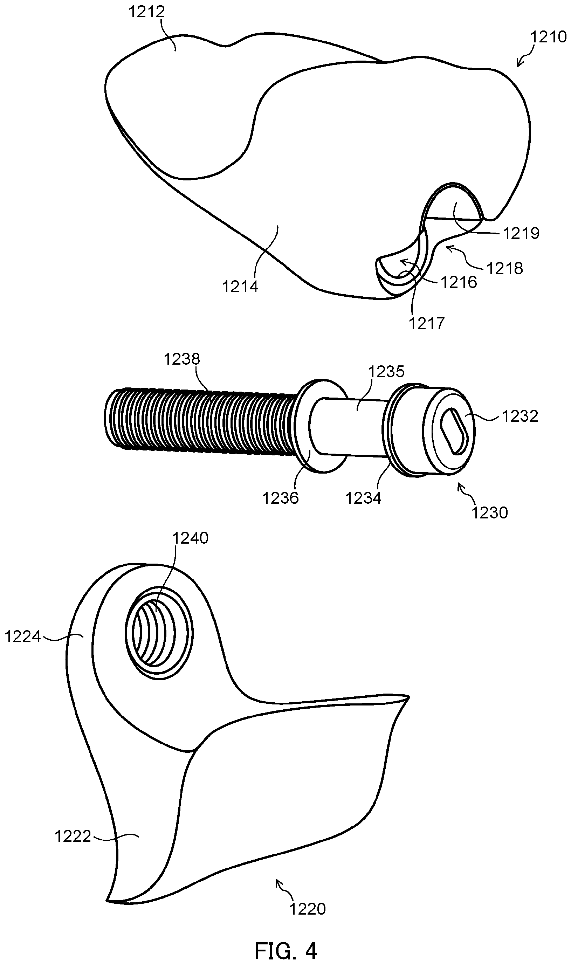

Lower-jaw advancing apparatus 1200 includes front-side retaining portion 1210 firmly fixed to upper-jaw oral appliance 1110, rear-side retaining portion 1220 firmly fixed to lower-jaw oral appliance 1120, and male screw 1230 and female screw 1240 that couple front-side retaining portion 1210 and rear-side retaining portion 1220 to each other such that front-side retaining portion 1210 and rear-side retaining portion 1220 are disposed with a predetermined interval therebetween as illustrated in FIG. 3 that is a schematic perspective view thereof and FIG. 4 that is an exploded perspective view thereof.

As illustrated in FIG. 2, lower-jaw advancing apparatus 1200 can position lower-jaw oral appliance 1120 to which rear-side retaining portion 1220 is firmly fixed frontward of upper-jaw oral appliance 1110 to which front-side retaining portion 1210 is firmly fixed by adjusting the screwing amount between male screw 1230 and female screw 1240 by screwdriver 1400 and positioning rear-side retaining portion 1220 with respect to front-side retaining portion 1210 in the front-rear direction.

As a result, lower-jaw advancing apparatus 1200 can limit the displacement of lower-jaw oral appliance 1120 to the rear side while moving lower-jaw oral appliance 1120 to the front side when the mouth is closed. As a result, oral appliance 1100 can suppress the respiratory arrest or hypopnea during sleep in a more effective manner by moving the lower jaw of the user wearing oral appliance 1100 to the front side and opening the trachea of the user in a larger manner.

Front-side retaining portion 1210 includes firmly fixed portion 1212 firmly fixed to upper-jaw oral appliance 1110, and front-side facing portion 1214 protruding from firmly fixed portion 1212 in the mouth-opening direction and disposed in a position that faces rear-side facing portion 1224 included in rear-side retaining portion 1220 when the mouth is closed. Retaining hole 1216 that retains male screw 1230 is formed in front-side facing portion 1214. Front-side retaining portion 1210 is disposed on downward facing surface 1114 corresponding to incisors of upper-jaw oral appliance 1110 (preferably, one pair of upper jaw medial incisors (dentition number 1)). By front-side facing portion 1214 protruding downward from downward facing surface 1114 corresponding to the incisors, the interval between each of the upper-jaw incisors and each of the lower-jaw incisors of the user wearing oral appliance 1100 is increased, and male screw 1230 is retained in the interval between the incisors.

Firmly fixed portion 1212 is disposed so as to cover downward facing surface 1114 corresponding to the incisors out of upper-jaw oral appliance 1110, and is firmly fixed to upper-jaw oral appliance 1110. From the viewpoint of enhancing the bond strength of front-side retaining portion 1210 with respect to upper-jaw oral appliance 1110, firmly fixed portion 1212 is preferred to cover downward facing surface 1114 of upper-jaw oral appliance 1110 so as to surround the incisors from the lip-side surface to the tongue-side surface through the lower jaw side.

Front-side facing portion 1214 is formed such that a part or the entirety of firmly fixed portion 1212 protrudes toward lower-jaw oral appliance 1120, and is disposed in a position that faces rear-side facing portion 1224 in the front-rear direction when the mouth is closed. In front-side facing portion 1214, retaining hole 1216 through which male screw 1230 is inserted is formed in a position facing female screw 1240 fixed to rear-side facing portion 1224 in the front-rear direction. The protruding length toward lower-jaw oral appliance 1120 only needs to be a length that enables retaining hole 1216 having a size through which engagement portion 1232 of male screw 1230 described below can be inserted to be formed in front-side facing portion 1214. However, from the viewpoint of suppressing the decrease in the wearing comfortability at the same time due to the interval between the incisors spreading too much, the protruding length is preferred to be 1.0 times or more and 2.5 times or less, further preferred to be 1.0 times or more and 2.0 times or less, and extremely preferred to be 1.0 times or more and 1.5 times or less with respect to the outer diameter of engagement portion 1232 of male screw 1230.

In front-side facing portion 1214, retaining hole 1216 that retains male screw 1230 by inserting cylindrical portion 1235 of male screw 1230 therethrough, and fitting portion 1218 in which engagement portion 1232 of male screw 1230 is disposed by being fitted therein on the front side of retaining hole 1216 are formed. Retaining hole 1216 is a through hole that is formed in front-side facing portion 1214 on the rear side thereof, and passes through front-side facing portion 1214 in the front-rear direction. Fitting portion 1218 is a dent formed on the front side of retaining hole 1216 out of front-side facing portion 1214, and having a shape that complements engagement portion 1232 of male screw 1230. From the viewpoint of reducing the discomfort of the user caused by male screw 1230 protruding to the front side of oral appliance 1100 and protruding male screw 1230 abutting against the lips or the inner sides of the cheeks, fitting portion 1218 is preferred to have a size that can accommodate engagement portion 1232 of male screw 1230. Specifically, fitting portion 1218 is preferred to have a width in the front-rear direction that is wider than the width of engagement portion 1232 of male screw 1230 in the front-rear direction when male screw 1230 is disposed in oral appliance 1100.

The surface serving as the outer periphery of retaining hole 1216 out of front-side facing portion 1214 may be protection surface 1217 covered with metal in order to suppress the wear and the like of front-side facing portion 1214 caused by the abutment with rotating male screw 1230. Similarly, the surface of fitting portion 1218 facing engagement portion 1232 of male screw 1230 may be protection surface 1219 covered with metal and the like in order to suppress the wear and the like of front-side facing portion 1214 caused by abutment with rotating male screw 1230.

Rear-side retaining portion 1220 includes protruding portion 1222 firmly fixed to lower-jaw oral appliance 1120 and disposed so as to protrude from the tongue-side surface of lower-jaw oral appliance 1120 to the rear side (tongue portion side), and flat-plate-like rear-side facing portion 1224 formed so as to extend upward in the rear end portion of protruding portion 1222 and disposed in a position facing front-side facing portion 1214 included in front-side retaining portion 1210 in the front-rear direction when the mouth is closed. On rear-side facing portion 1224, female screw 1240 is fixed. In rear-side retaining portion 1220, the distal end of protruding portion 1222 is firmly fixed to the tongue-side surface of lower-jaw oral appliance 1120 corresponding to the incisors (preferably one pair of lower-jaw medial incisors (dentition number 1)), and female screw 1240 is retained in a space on the tongue side than oral appliance 1100 by rear-side facing portion 1224 extending upward from the rear end portion of protruding portion 1222 protruding to the rear side from the tongue-side surface corresponding to the incisors described above.

Protruding portion 1222 has one end fixed to the tongue-side surface of lower-jaw oral appliance 1120, and disposes rear-side facing portion 1224 behind the dentition by protruding to the tongue portion side. From the viewpoint of enhancing the bond strength of rear-side retaining portion 1220 with respect to lower-jaw oral appliance 1120, protruding portion 1222 may have one end fixed to lower-jaw oral appliance 1120 by covering downward facing surface 1114 of lower-jaw oral appliance 1120 so as to surround the incisors from the front side (lip-side surface) to the rear side (tongue-side surface) through the lower jaw side. However, from the viewpoint of reducing the mouth-opening amount and increasing the wearing comfortability of the user wearing oral appliance 1100, protruding portion 1222 is preferred to be fixed to lower-jaw oral appliance 1120 by coming into contact with only the tongue-side surface of lower-jaw oral appliance 1120.

Protruding portion 1222 protrudes to the rear side from the tongue-side surface of the lower-jaw oral appliance in a position below upward facing surface 1124 of lower-jaw oral appliance 1120 in a side view. In this embodiment, protruding portion 1222 has a curved shape in which the upper surface thereof gently falls from lower-jaw oral appliance 1120 on the front side, and gently rises toward rear-side facing portion 1224 on the rear side. With the shape in which rear-side facing portion 1224 is continuously formed from protruding portion 1222, the abutment pressure from the tongue of the user is dispersed and the damage of rear-side retaining portion 1220 is suppressed.

Rear-side facing portion 1224 is disposed in a position facing front-side facing portion 1214 in the front-rear direction when the mouth is closed. In rear-side facing portion 1224, female screw 1240 is fixed in a position that faces retaining hole 1216 in front-side facing portion 1214 when the mouth is closed. As a result, male screw 1230 and female screw 1240 are disposed in a height between downward facing surface 1114 of upper-jaw oral appliance 1110 and upward facing surface 1124 of lower-jaw oral appliance 1120 in a side view of oral appliance 1100.

The thickness of rear-side facing portion 1224 to the front-rear direction only needs to be equal to or more than the length of female screw 1240 in the front-rear direction when female screw 1240 is disposed in oral appliance 1100. However, from the viewpoint of suppressing the decrease in the wearing comfortability, the thickness is preferred to be 1.0 times or more and 2.5 times or less, further preferred to be 1.0 times or more and 2.0 times or less, and extremely preferred to be 1.0 times or more and 1.5 times or less with respect to the length of female screw 1240 in the front-rear direction described above.

Male screw 1230 includes engagement portion 1232 engageable with screwdriver 1400, front-side locking portion 1234 and rear-side locking portion 1236, ring-shaped cylindrical portion 1235 disposed between front-side locking portion 1234 and rear-side locking portion 1236, and screw portion 1238 that can be threaded into female screw 1240. Engagement portion 1232, front-side locking portion 1234, cylindrical portion 1235, rear-side locking portion 1236, and screw portion 1238 are disposed from one distal end of male screw 1230 to the other distal end thereof in the order presented.

Engagement portion 1232 is disposed on a front-side end of male screw 1230 or between the front-side end and front-side locking portion 1234. Engagement portion 1232 only needs to have a shape that is engageable with screwdriver 1400 and enables male screw 1230 to be rotated by rotating engaged screwdriver 1400. For example, engagement portion 1232 can be a hexagon socket and have a shape that enables male screw 1230 to be rotated by inserting a distal end of hexagonal-prism-shaped screwdriver 1400 (hex key) that can be fitted in the hexagon socket therein. Alternatively, engagement portion 1232 can be an elliptic-cylindrical socket, and can have a shape that enables male screw 1230 to be rotated by inserting a distal end of an elliptic-cylindrical screwdriver that can be fitted in the elliptic-cylindrical socket therein.

Front-side locking portion 1234, cylindrical portion 1235, and rear-side locking portion 1236 rotatably fix the position of male screw 1230 with respect to front-side retaining portion 1210 when male screw 1230 is inserted through retaining hole 1216 formed in front-side facing portion 1214 of front-side retaining portion 1210.

Specifically, front-side locking portion 1234 is a substantially-U-shaped thin-plate-like member formed in a larger shape than retaining hole 1216, and is fitted on a front-side narrow portion formed in cylindrical portion 1235 on the front side thereof and mounted around the front-side narrow portion. Rear-side locking portion 1236 is a substantially-U-shaped thin-plate-like member formed in a shape larger than retaining hole 1216, and is fitted on a rear-side narrow portion formed in cylindrical portion 1235 on the rear side thereof and mounted around the rear-side narrow portion. As a result, male screw 1230 is formed as follows. In front-side locking portion 1234 and rear-side locking portion 1236, the radius of the outer periphery thereof is formed to be larger than the radius of retaining hole 1216. In cylindrical portion 1235, the radius of the outer periphery thereof is formed to be smaller than the radius of retaining hole 1216. Therefore, the movement of male screw 1230 with respect to front-side retaining portion 1210 in the front-rear direction may be limited in the interval between front-side locking portion 1234 and rear-side locking portion 1236, and the position of male screw 1230 may be rotatably fixed with respect to front-side retaining portion 1210.

Screw portion 1238 is disposed on the rear-side portion of male screw 1230. Screw portion 1238 only needs to have a screw shape that can be threaded into female screw 1240. From the viewpoint of increasing the advance amount of lower-jaw oral appliance 1120 with respect to upper-jaw oral appliance 1110 by increasing the advance amount of rear-side retaining portion 1220 with respect to front-side retaining portion 1210, screw portion 1238 is preferred to have a length that is half of male screw 1230 or more.

Female screw 1240 is fixed to rear-side facing portion 1224 of rear-side retaining portion 1220 so as to face retaining hole 1216 in front-side retaining portion 1210. As a result, female screw 1240 is threaded onto male screw 1230 behind the dentition surface in the upper-jaw oral appliance and the lower-jaw oral appliance.

According to oral appliance 1100 having the configuration described above, by screwing screw portion 1238 of male screw 1230 retained by upper-jaw oral appliance 1110 by front-side retaining portion 1210 together with female screw 1240 retained by lower-jaw oral appliance 1120 by rear-side retaining portion 1220, rear-side retaining portion 1220 is coupled to front-side retaining portion 1210 and lower-jaw oral appliance 1120 is coupled to upper-jaw oral appliance 1110. By adjusting the screwing amount between screw portion 1238 of male screw 1230 and female screw 1240, rear-side retaining portion 1220 is positioned with respect to front-side retaining portion 1210 in the front-rear direction. As a result, lower-jaw oral appliance 1120 to which rear-side retaining portion 1220 is firmly fixed can also be positioned on the front side with respect to upper-jaw oral appliance 1110 to which front-side retaining portion 1210 is firmly fixed.

At this time, by adjusting the screwing amount between screw portion 1238 of male screw 1230 and female screw 1240, as appropriate, the advance amount of lower-jaw oral appliance 1120 with respect to upper-jaw oral appliance 1110 can be adjusted. As a result, by moving lower-jaw oral appliance 1120 frontward of upper-jaw oral appliance 1110 by the amount fitted for the shape of the jaw of the user, the trachea of the user wearing oral appliance 1100 can be caused to become less likely to be constricted.

According to oral appliance 1100 having the configuration described above, male screw 1230 is disposed from and behind the dentition surface in the upper-jaw oral appliance and the lower-jaw oral appliance. Therefore, the upsizing of the oral appliance caused by male screw 1230 being disposed frontward of the dentition surface, and the decrease of the wearing comfortability of the user caused by male screw 1230 abutting against the lips and male screw 1230 causing the closing of the mouth with lips closed together to be difficult, for example, are suppressed.

Upper-jaw oral appliance 1110 and lower-jaw oral appliance 1120 are made of a material that is hardly damaged by a normal occlusal force of a person.

For example, upper-jaw oral appliance 1110 and lower-jaw oral appliance 1120 may be made of a single hard material (for example, acrylic resin) of which Flexural modulus measured according to JIS T 6501 is 2000 MPa or more and 3000 MPa or less, or a material obtained by combining a soft material that is 10 MPa or more and 300 MPa or less and a hard material that is 1000 MPa or more and 3000 MPa or less.

Upper-jaw oral appliance 1110 and lower-jaw oral appliance 1120 may be made of a relatively soft material of which tensile strength is 150 N or more and less than 2000 N, preferably 150 N or more and 500 N or less in order to increase the followability with respect to the teeth of the user.

Note that the tensile strength means the strength by which an upper-jaw oral appliance of an oral appliance (thickness of 3 mm) manufactured with use of a standard model of Nissin Dental Products INC. is torn when a hole of ∅1.5 mm is opened in the dentition number 6 in the upper-jaw oral appliance described above and tensile testing to the molar direction (rear side) is performed.

Examples of materials of which tensile strength is 150 N or more and less than 2000 N include olefin-based resin, polyester-based resin, urethane-based resin, polyamide-based resin, and acrylic rubber resin. Out of the above, olefin-based resin is preferred.

The olefin-based resin is a polymer obtained by homo-polymerizing olefin, or a copolymer obtained by polymerizing olefin and another monomer. As olefin, olefin of which carbon number is 2 to 6 including ethylene, propylene, butene, methyl-pentene, and hexene is preferred. Examples of other monomers include vinyl acetate.

As the olefin-based resin, polyethylene (PE), polyethylene-based resin, polypropylene (PP), polypropylene-based resin, ethylene-vinyl acetate copolymer (EVA), and the like are preferred, and polyethylene(PE), polyethylene-based resin, polypropylene (PP), polypropylene-based resin, and the like are more preferred.

The polyester-based resin is a poly-condensate of multivalent carboxylic acid (dicarboxylic acid and the like) and polyalcohol (diol and the like). Examples of the polyester-based resin include polyethylene-terephthalate (PET).

The urethane-based resin is a poly-condensate of a compound containing isocyanate groups and a compound containing hydroxyl groups. Examples of the urethane-based resin include thermoplastic polyurethane (TPU).

The polyamide-based resin is a (co)polymer obtained by bonding a large number of monomers together by amide bond. Examples of the polyamide-based resin include nylon, para-amide, and meta-amide.

The acrylic rubber resin is a (co)polymer of which main component is acrylic rubber. Examples of the acrylic resin include a block copolymer of methyl methacrylate and butyl acrylate.

As the material of which tensile strength is 150 N or more and less than 2000 N, commercially available materials such as F327 (polypropylene-based resin) made by Prime Polymer Co., Ltd. may be used.

Front-side retaining portion 1210 and rear-side retaining portion 1220 can be made of a hard resin material (for example, polycarbonate resin) of which flexural modulus measured according to JIS T 6501 is 1000 MPa or more and 3000 MPa or less, metal materials such as titanium or an alloy containing titanium, iron (steel including stainless steel and the like), and alloys of gold, silver, platinum, cobalt, chromium, and the like, and the like. Note that the corrosion resistance of the metal materials described above can be increased by chemical conversion coating and the like.

Male screw 1230 and female screw 1240 can be made of metal materials such as titanium or an alloy containing titanium, iron (steel including stainless steel and the like), and alloys of gold, silver, platinum, cobalt, chromium, and the like, a hard resin material (for example, polycarbonate resin) of which flexural modulus measured according to JIS T 6501 is 1000 MPa or more and 3000 MPa or less, and the like. Note that the corrosion resistance of the metal materials described above may be increased by chemical conversion coating and the like.

For example, front-side retaining portion 1210 can be caused to retain male screw 1230 by causing a photocurable material to flow into a mold for front-side retaining portion 1210 in which male screw 1230 is disposed, and curing the photocurable material by irradiating the photocurable material with an ultraviolet ray and the like. Alternatively, front-side retaining portion 1210 can be caused to retain male screw 1230 by forming front-side retaining portion 1210 by the resin or the metal described above and inserting male screw 1230 through retaining hole 1216, and then fitting front-side locking portion 1234 on the front-side narrow portion and fitting rear-side locking portion 1236 on the rear-side narrow portion.

Front-side retaining portion 1210 may be manufactured separately from upper-jaw oral appliance 1110 with a method described above, for example, and may be caused to adhere to upper-jaw oral appliance 1110. Alternatively, front-side retaining portion 1210 can be fixed to upper-jaw oral appliance 1110 by causing a photocurable material to flow into a mold for upper-jaw oral appliance 1110 in which front-side retaining portion 1210 is disposed and curing the photocurable material by irradiating the photocurable material with an ultraviolet ray and the like.

Rear-side retaining portion 1220 can be caused to retain female screw 1240 by, for example, causing a photocurable material to flow into a mold for front-side retaining portion 1210 in which female screw 1240 is disposed and curing the photocurable material by irradiating the photocurable material with an ultraviolet ray and the like. Alternatively, rear-side retaining portion 1220 may be caused to retain female screw 1240 by fitting female screw 1240 in rear-side retaining portion 1220 after rear-side retaining portion 1220 is manufactured in a shape having a dent or a through hole in a shape that can retain female screw 1240 separately from female screw 1240. Alternatively, rear-side retaining portion 1220 may be integrally molded with female screw 1240 by the same material as female screw 1240.

Rear-side retaining portion 1220 may be manufactured separately from lower-jaw oral appliance 1120 with a method described above, for example, and may be caused to adhere to lower-jaw oral appliance 1120. Alternatively, rear-side retaining portion 1220 can be fixed to lower-jaw oral appliance 1120 by causing a photocurable material to flow into a mold for lower-jaw oral appliance 1120 in which rear-side retaining portion 1220 is disposed, and curing the photocurable material by irradiating the photocurable material with an ultraviolet ray and the like.

### (Effect)

According to this embodiment, the advance amount of the lower-jaw oral appliance can be adjusted in an easier manner without upsizing the oral appliance.

### [Embodiment 2]

An oral appliance according to Embodiment 2 of the present invention is different from oral appliance 1100 according to Embodiment 1 in that the front-side retaining portion and the rear-side retaining portion retain the male screw such that the male screw is displaceable to the left and the right or turnable in the left-right direction in the oral appliance according to Embodiment 2. Therefore, overlapping description is omitted, and only the configuration of front-side retaining portion 1210 and rear-side retaining portion 1220 different from oral appliance 1100 according to Embodiment 1 is described below.

### (First Configuration of Front-side Retaining Portion)

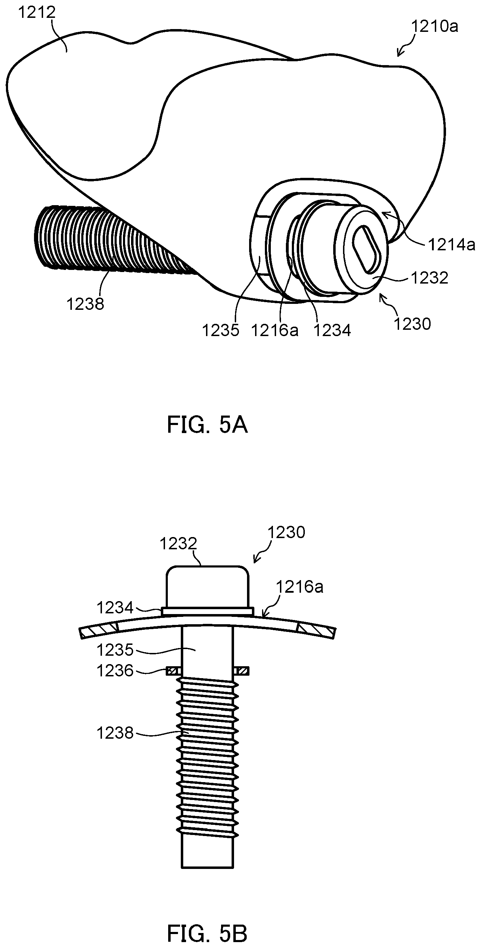

FIG. 5A is a perspective view schematically illustrating a first configuration of the front-side retaining portion in this embodiment, and FIG. 5B is a cross-sectional plan view schematically illustrating the first configuration described above. In order to facilitate the description, male screw 1230 is also illustrated in FIG. 5A and FIG. 5B.

In front-side retaining portion 1210a according to this configuration example, retaining hole 1216a formed in front-side facing portion 1214a is a substantially-elliptic through hole formed to be wide-width on the left and the right. Note that, when a protection surface is formed on retaining hole 1216a, the protection surface is also naturally substantially elliptic-cylindrical. As a result, front-side retaining portion 1210a retains male screw 1230 such that male screw 1230 is displaceable to the left and the right or turnable in the left-right direction by inserting cylindrical portion 1235 of male screw 1230 through retaining hole 1216a.

### (Second Configuration of Front-side Retaining Portion)

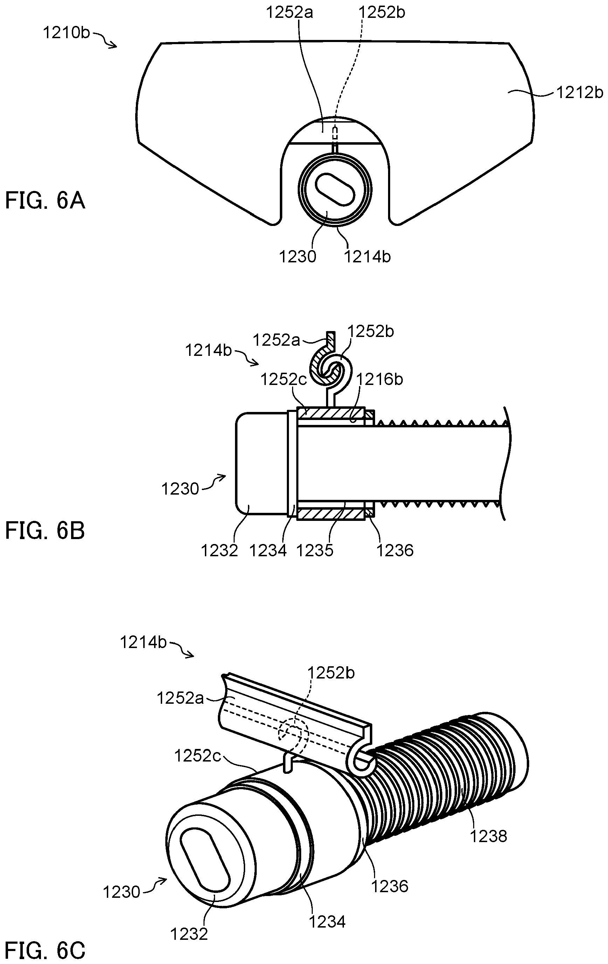

FIG. 6A is a front view schematically illustrating a second configuration of the front-side retaining portion in this embodiment, FIG. 6B is a cross-sectional view schematically illustrating the second configuration described above, and FIG. 6C is a partial perspective view schematically illustrating the second configuration described above. In order to facilitate the description, male screw 1230 is also illustrated in FIG. 6A, FIG. 6B, and FIG. 6C.

In front-side retaining portion 1210b according to this configuration example, front-side facing portion 1214b includes guide hook 1252a extending in the left-right direction on the lower end portion of firmly fixed portion 1212b, and sliding member 1252c retained by guide hook 1252a. Sliding member 1252c is attached to guide hook 1252a so as to be movable in the left-right direction by hooking hooking hook 1252b fixed to the upper end thereof on guide hook 1252a. Front-side retaining portion 1210b includes hollow-cylindrical retaining hole 1216b formed in sliding member 1252c, and retains male screw 1230 such that male screw 1230 is displaceable to the left and the right or turnable in the left-right direction by inserting cylindrical portion 1235 of male screw 1230 through retaining hole 1216b and fixing the position of male screw 1230 with respect to sliding member 1252c.

### (Third Configuration of Front-side Retaining Portion)

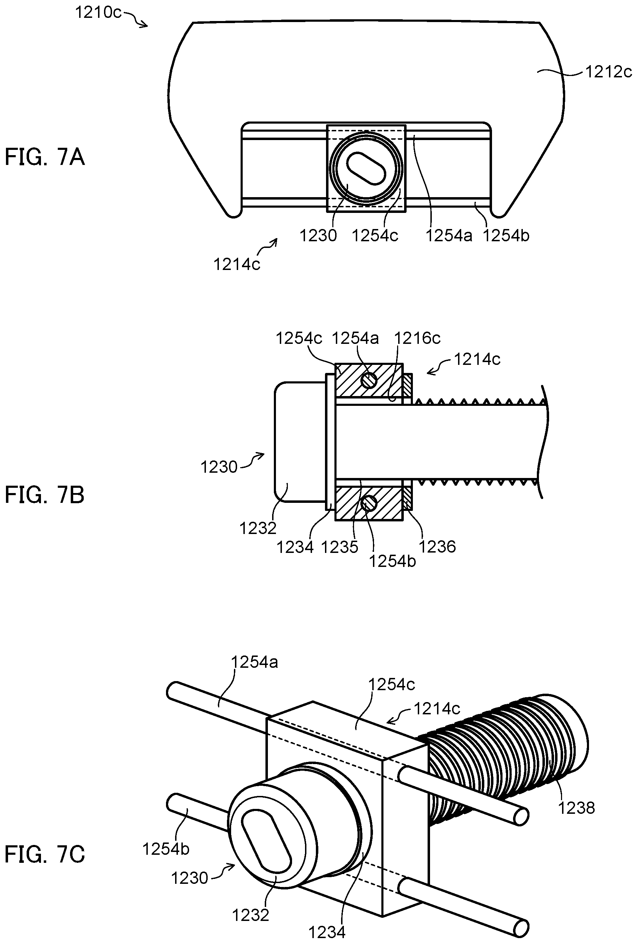

FIG. 7A is a front view schematically illustrating a third configuration of the front-side retaining portion in this embodiment, FIG. 7B is a cross-sectional view schematically illustrating the third configuration described above, and FIG. 7C is a partial perspective view schematically illustrating the third configuration described above. In order to facilitate the description, male screw 1230 is also illustrated in FIG. 7A, FIG. 7B, and FIG. 7C.

In front-side retaining portion 1210c according to this configuration example, front-side facing portion 1214c includes two guide bars, that is, guide bar 1254a and guide bar 1254b disposed in different heights and extending in the left-right direction on the lower end portion of firmly fixed portion 1212c, and sliding member 1254c retained by guide bar 1254a and guide bar 1254b. Sliding member 1254c is mounted so as to be moveable in the left-right direction along the two guide bars, that is, guide bar 1254a and guide bar 1254b by inserting guide bar 1254a and guide bar 1254b through sliding member 1254c. Front-side retaining portion 1210c includes hollow-cylindrical retaining hole 1216c formed in sliding member 1254c, and retains male screw 1230 such that male screw 1230 is displaceable to the left and the right or turnable in the left-right direction by inserting cylindrical portion 1235 of male screw 1230 through retaining hole 1216c and fixing the position of male screw 1230 with respect to sliding member 1254c.

### (Fourth Configuration of Front-side Retaining Portion)

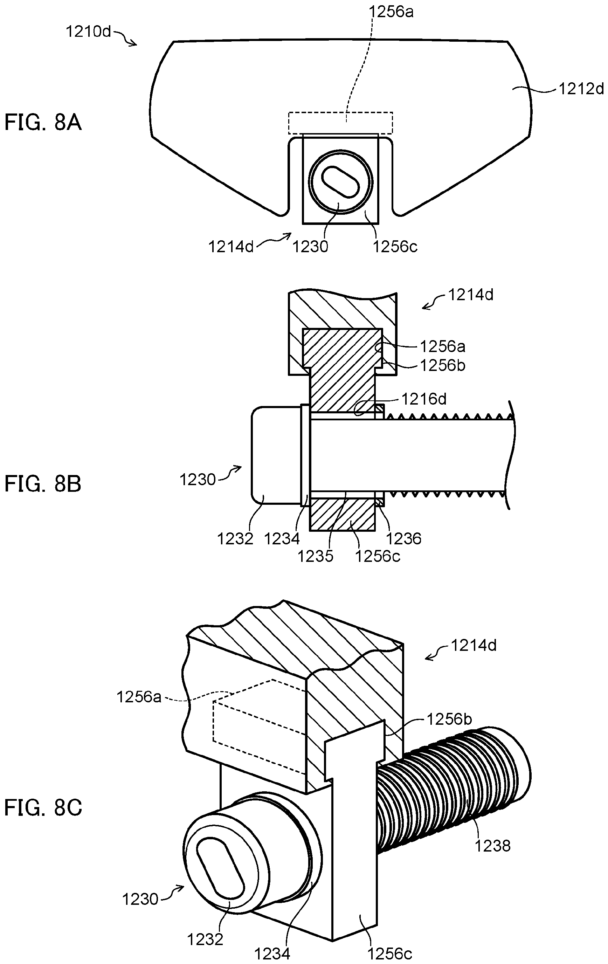

FIG. 8A is a front view schematically illustrating a fourth configuration of the front-side retaining portion in this embodiment, FIG. 8B is a cross-sectional view schematically illustrating the fourth configuration described above, and FIG. 8C is a partial perspective view schematically illustrating the fourth configuration described above. In order to facilitate the description, male screw 1230 is also illustrated in FIG. 8A, FIG. 8B, and FIG. 8C.

In front-side retaining portion 1210d according to this configuration example, front-side facing portion 1214d includes guide rail 1256a, which is a hollow-like space extending in the left-right direction formed in the lower end portion of firmly fixed portion 1212d and has an opening extending in the left-right direction formed on the lower end side, and sliding member 1256c retained by guide rail 1256a. Sliding member 1256c is attached to guide rail 1256a so as to be moveable in the left-right direction by hooking hammer-like hammer portion 1256b continuously formed from the upper end thereof on guide rail 1256a. At this time, guide rail 1256a is formed such that the width of the hollow-like space formed in the lower end portion of firmly fixed portion 1212d in the front-rear direction is larger than the width of the opening toward the lower end side in the front-rear direction, and the width of hammer portion 1256b is increased in the front-rear direction such that the upper end portion thereof fits in the hollow-like space formed in the lower end portion of firmly fixed portion 1212d. As a result, hammer portion 1256b is moveable in the left-right direction along guide rail 1256a. Front-side retaining portion 1210d includes hollow-cylindrical retaining hole 1216d formed in sliding member 1256c, and retains male screw 1230 such that male screw 1230 is displaceable to the left and the right or turnable in the left-right direction by inserting cylindrical portion 1235 of male screw 1230 through retaining hole 1216d and fixing the position of male screw 1230 with respect to sliding member 1256c.

### (First Configuration of Rear-side Retaining Portion)

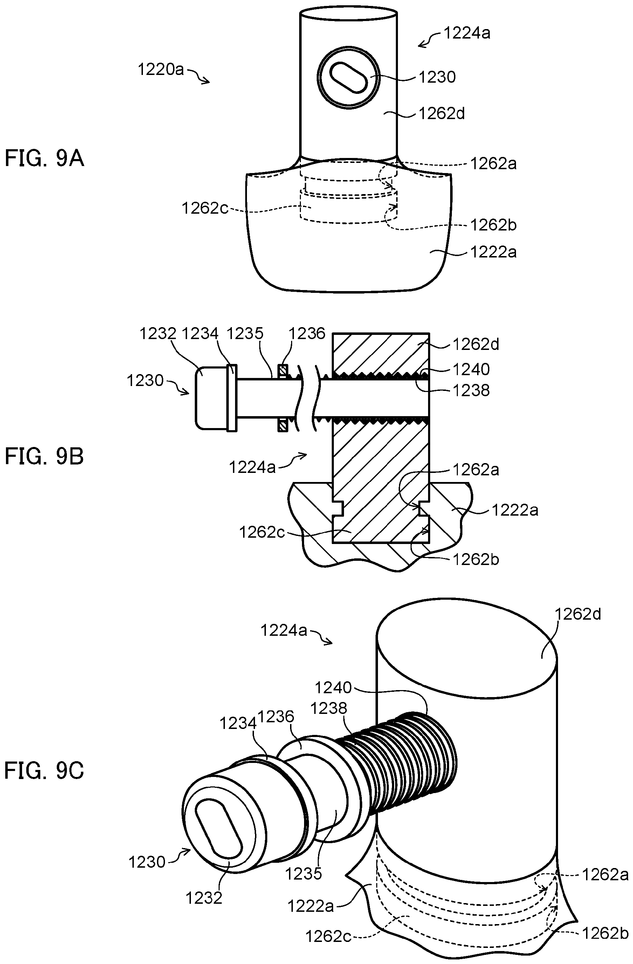

FIG. 9A is a front view schematically illustrating a first configuration of the rear-side retaining portion in this embodiment, FIG. 9B is a cross-sectional view schematically illustrating the first configuration described above, and FIG. 9C is a partial perspective view schematically illustrating the first configuration described above. In order to facilitate the description, male screw 1230 and female screw 1240 are also illustrated in FIG. 9A, FIG. 9B, and FIG. 9C.

Rear-side retaining portion 1220a according to this configuration example includes turn retaining hole 1262a. Turn retaining hole 1262a is opened in the inner portion of the rear end portion of protruding portion 1222a to the upper end side, and includes a hook portion in which the opening diameter is reduced between the opening and the bottom surface. Rear-side facing portion 1224a includes stopper 1262c, which is inserted in turn retaining hole 1262a and includes diameter-reduced portion 1262b having a shape that can be hooked on the hook portion, and turn retaining portion 1262d, which is continuously formed upward from stopper 1262c and fixes female screw 1240 at a portion outside turn retaining hole 1262a. Rear-side retaining portion 1220a retains female screw 1240 fixed to turn retaining portion 1262d such that female screw 1240 is turnable by inserting stopper 1262c in turn retaining hole 1262a such that stopper 1262c is turnable, and fixing the position of turn retaining portion 1262d such that turn retaining portion 1262d is turnable by regulating the displacement of the turn retaining portion to the up-down direction by diameter-reduced portion 1262b. By retaining female screw 1240 by rear-side retaining portion 1220a such that female screw 1240 is turnable as above, male screw 1230 threaded into female screw 1240 also becomes turnable in the left-right direction.

Rear-side retaining portion 1220a according to the first configuration described above may be used in combination with any of the first configuration, the second configuration, the third configuration, and the fourth configuration of the front-side retaining portion.

Note that, in this configuration, turn retaining hole 1262a may include a hook portion in which the opening diameter is enlarged instead of the hook portion in which the opening diameter is reduced, and rear-side facing portion 1224a may include a diameter-enlarged portion in which the opening diameter is enlarged instead of a diameter-reduced portion that can be hooked on the hook portion.

### (Second Configuration of Rear-side Retaining Portion)

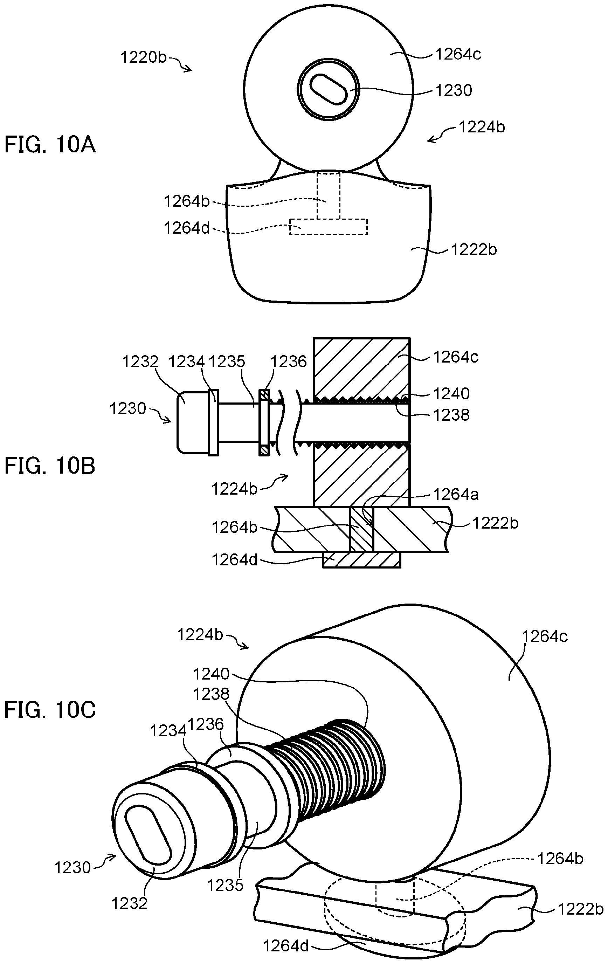

FIG. 10A is a front view schematically illustrating a second configuration of the rear-side retaining portion in this embodiment, FIG. 10B is a cross-sectional view schematically illustrating the second configuration described above, and FIG. 10C is a partial perspective view schematically illustrating the second configuration described above. In order to facilitate the description, male screw 1230 and female screw 1240 are also illustrated in FIG. 10A, FIG. 10B, and FIG. 10C.

In rear-side retaining portion 1220b according to this configuration example, a rear end portion of protruding portion 1222b has a thin-plate-like shape extending to the front-rear direction, and through hole 1264a that passes through the rear end portion having the thin-plate-like shape in the up-down direction is formed. Rear-side facing portion 1224b includes insertion bar 1264b inserted through through hole 1264a so as to be turnable, stopper 1264c, and turn retaining portion 1264d that retains female screw 1240 such that female screw 1240 is turnable. Stopper 1264c and turn retaining portion 1264d both have a shape with a wider width than through hole 1264a. Rear side facing portion 220b retains female screw 1240 fixed to turn retaining portion 1264d such that female screw 1240 is turnable by inserting insertion bar 1264b in turn retaining hole 1264a such that insertion bar 1264b is turnable, and fixing the position of turn retaining portion 1264d such that turn retaining portion 1264d is turnable by regulating the displacement of turn retaining portion 1264d to the up-down direction by stopper 1264c. By retaining female screw 1240 by rear-side retaining portion 1220b such that female screw 1240 is turnable as above, male screw 1230 threaded into female screw 1240 also becomes turnable in the left-right direction.

Rear-side retaining portion 1220b according to the second configuration described above may be used in combination with any of the first configuration, the second configuration, the third configuration, and the fourth configuration of the front-side retaining portion.

### (Third Configuration of Rear-side Retaining Portion)

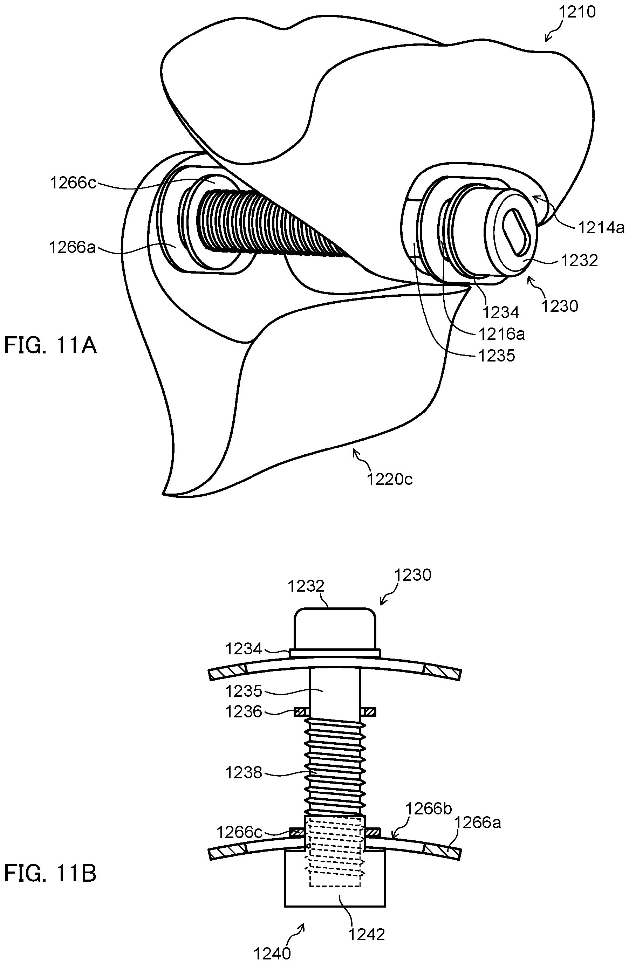

FIG. 11A is a perspective view schematically illustrating a third configuration of the rear-side retaining portion in this embodiment, and FIG. 11B is a cross-sectional plan view schematically illustrating the third configuration described above. In order to facilitate the description, front-side retaining portion 1210, male screw 1230, and female screw 1240 are also illustrated in FIG. 11A and FIG. 11B.

Rear-side retaining portion 1220c according to this configuration example retains female screw 1240 such that female screw 1240 is displaceable to the left and the right by inserting female screw 1240 through substantially-elliptic rear-side retaining hole 1266b that is wide-width to the left and the right formed in rear-side retaining portion 1266a. Meanwhile, rear-side retaining portion 1220c limits the movement of female screw 1240 to the front-rear direction by female screw rear end portion 1242, which is formed with a wider width than rear-side retaining hole 1266b and cannot pass through rear-side retaining hole 1266b, and female screw locking portion 1266c, which is fixed to female screw 1240 and locks female screw 1240 on the front side of rear-side retaining hole 1266b. Rear-side retaining portion 1220c according to the third configuration described above may be used in combination with any of the first configuration, the second configuration, the third configuration, and the fourth configuration of the front-side retaining portion, but is preferred to be combined with the first configuration of the front-side retaining portion as illustrated in FIG. 11A and FIG. 11B from the viewpoint of simplifying the configuration of front-side retaining portion 1210 and rear-side retaining portion 1220.

### (Fourth Configuration of Rear-side Retaining Portion)

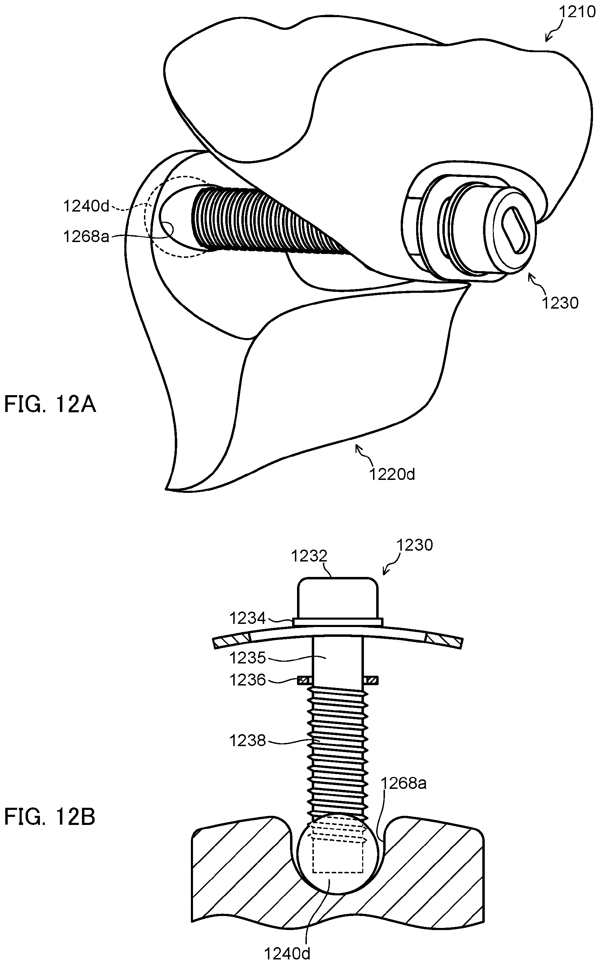

FIG. 12A is a perspective view schematically illustrating a fourth configuration of the rear-side retaining portion in this embodiment, and FIG. 12B is a cross-sectional plan view schematically illustrating the fourth configuration described above. In order to facilitate the description, front-side retaining portion 1210, male screw 1230 and female screw 1240 are also illustrated in FIG. 12A and FIG. 12B.

In this configuration example, female screw 1240d is a substantially spherical female screw, and rear-side retaining portion 1220d has spherical female screw hole 1268a that is opened to the distal end side and accommodates substantially spherical female screw 1240d. Female screw hole 1268a has an opening having a width (for example, substantially the same width as the outer diameter of male screw 1230) that is smaller than the diameter of female screw 1240d in the up-down direction and a wider width in the left-right direction in an end surface on front-side retaining portion 1210 side. As a result, male screw 1230 threaded into female screw 1240d also becomes turnable in the left-right direction. Rear-side retaining portion 1220d according to the fourth configuration described above may be used in combination with any of the first configuration, the second configuration, the third configuration, and the fourth configuration of the front-side retaining portion.

### (Effects)

According to this embodiment, male screw 1230 is displaceable or turnable in the left-right direction, and hence the position of lower-jaw oral appliance 1120 is not fixed with respect to upper-jaw oral appliance 1110, and lower-jaw oral appliance 1120 can also be displaced or turn in the left-right direction with respect to upper-jaw oral appliance 1110. Therefore, the user wearing the oral appliance according to this embodiment can displace the lower jaw to the left-right direction while wearing the oral appliance. As a result, the oral appliance according to this embodiment can reduce the discomfort of the user caused by the position of the lower jaw being fixed to the upper jaw. The upper-jaw oral appliance and the lower-jaw oral appliance can also be prevented from being damaged by the force in the left-right direction due to bruxism and the like.

### [Embodiment 3]

Oral appliance 1300 according to Embodiment 3 of the present invention is different from the oral appliance according to Embodiment 1 or the oral appliance according to Embodiment 2 in that the rear-side retaining portion includes a pressing down portion that presses down the tongue portion of the user when the mouth is closed in oral appliance 1300 according to Embodiment 3. Therefore, overlapping description is omitted, and only the configuration of rear-side retaining portion that is different from the oral appliance according to Embodiment 1 or the oral appliance according to Embodiment 2 is described below.

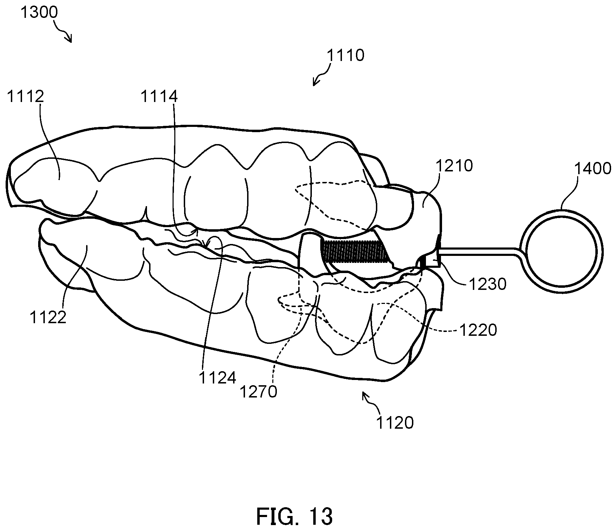

FIG. 13 is a side view schematically illustrating the configuration of oral appliance 1300 according to this embodiment. In oral appliance 1300, rear-side retaining portion 1220 includes pressing down portion 1270.

Pressing down portion 1270 is spatula-like member extending to the rear side from the rear-side retaining portion, and presses down the tongue portion of the user when the mouth is closed. When pressing down portion 1270 presses down the tongue portion of the user wearing oral appliance 1300, the trachea of the user is opened in an easier manner.

Note that the shape of pressing down portion 1270 is not particularly limited, and may extend to both of the left-right direction and the rear side, for example.

### (Effects)

According to this embodiment, by pressing down the tongue portion of the user wearing oral appliance 1300 by pressing down portion 1270, the trachea of the user is opened in an easier manner. Therefore, the constriction of the trachea of the user wearing oral appliance 1300 can be prevented, and the respiratory arrest or hypopnea during sleep can be suppressed.

### [Modified Examples of Embodiment 1 to Embodiment 3]

Note that each of Embodiment 1 to Embodiment 3 describes one example of a first aspect of the present invention, and it goes without saying that the first aspect of the present invention is not limited to the embodiments described above, and other various embodiments are also possible within the scope of the idea of the present invention.

For example, in Embodiment 1 to Embodiment 3, the female screw is integrally molded with the rear-side retaining portion or is bonded to the rear-side retaining portion, but the female screw may be removably retained by the rear-side retaining portion. Meanwhile, in Embodiment 1 to Embodiment 3, the male screw is removably retained by the front-side retaining portion, but the male screw may be fixed to the front-side retaining portion in a rotatable manner.

In Embodiment 1 to Embodiment 3, the front-side retaining portion retaining the male screw is fixed to the upper-jaw oral appliance, and the rear-side retaining portion retaining the female screw is fixed to the lower-jaw oral appliance. However, the front-side retaining portion retaining the male screw may be fixed to the lower-jaw oral appliance, and the rear-side retaining portion retaining the female screw may be fixed to the upper-jaw oral appliance. At this time, by setting the rotation direction of the male screw when the male screw is threaded into the female screw to be in the opposite direction, the lower-jaw oral appliance can be moved frontward of the upper-jaw oral appliance by the rotation of the male screw.

Alternatively, the front-side retaining portion retaining the male screw may be fixed to the upper-jaw oral appliance or the lower-jaw oral appliance, and the rear-side retaining portion retaining the male screw may be fixed to the lower-jaw oral appliance or the upper-jaw oral appliance. At this time, the female screw may be threaded onto the male screw at the dentition surfaces of the upper-jaw oral appliance and the lower-jaw oral appliance. At this time, the direction of the male screw is opposite to Embodiment 1 to Embodiment 3, and the engagement portion of the male screw is disposed so as to face the rear side of the oral appliance. At this time, for example, in Embodiment 2, the male screw retained by the rear-side retaining portion may be turnable or displaceable in the left-right direction, and the female screw retained by the front-side retaining portion may be displaced in the left-right direction.

In Embodiment 1 to Embodiment 3, the front-side retaining portion is disposed between the dentitions of the upper-jaw oral appliance and the lower-jaw oral appliance, but both of the front-side retaining portion and the rear-side retaining portion may be disposed behind the dentition surfaces of the upper-jaw oral appliance and the lower-jaw oral appliance.

In Embodiment 1 to Embodiment 3, the torque for the engagement of the male screw and the female screw may be partially different in the male screw or the female screw.

For example, the screw portion of the male screw may have two types of outer diameters in which the outer diameter on the distal end portion side on the rear side is larger than the outer diameter on the collar portion side, or may have a shape in which the outer diameter continuously increases from the collar portion side to the distal end portion side on the rear side. Alternatively, in the screw portion of the male screw, the outer diameter of the distal end portion on the rear side may be enlarged so as to extend to the outer side by providing a gap portion or a slit from the distal end on the rear side in collar portion direction. The outer diameter of the distal end portion on the rear side can be increased by opening the distal end portion on the rear side to the outer side by changing the width of the slit in the depth direction such that the width of the distal end portion on the rear side increases. Alternatively, the female screw may have two types of inner diameters in which the inner diameter on the front side is smaller than the inner diameter on the rear side, or may have a shape in which the inner diameter continuously decreases from the rear side to the front side.

Alternatively, the screw portion of the male screw or the female screw may have a small-pitch portion in which the pitch of the screw partially decreases. The small-pitch portion may have a pitch with a fixed width that is smaller than other regions, or may have a pitch that continuously decreases toward the front side or the rear side.

In Embodiment 1 to Embodiment 3, the dentition impression portions that can be worn on the dentition are formed in the upper-jaw oral appliance main-body portion and the lower-jaw oral appliance main-body portion. However, the upper-jaw oral appliance main-body portion and the lower-jaw oral appliance main-body portion do not necessarily need to include the dentition impressions as long as the upper-jaw oral appliance main-body portion and the lower-jaw oral appliance main-body portion each have a shape in which the dentition fit, and may include the dentition impression portions in only a part of the upper-jaw oral appliance main-body portion and the lower-jaw oral appliance main-body portion.

The upper-jaw oral appliance and the lower-jaw oral appliance may be formed by a plurality of materials, and may be made by combining a portion formed by an organic matter and a portion formed by an inorganic matter.

### [Configuration Example of Oral Appliance according to First Aspect]

According to the first aspect of the present invention, there is provided an oral appliance, including: one pair of an upper-jaw oral appliance and a lower-jaw oral appliance facing each other; a male screw; a female screw; a front-side retaining portion that is disposed on one oral appliance out of the upper-jaw oral appliance and the lower-jaw oral appliance and retains the male screw or the female screw; and a rear-side retaining portion that is disposed on another oral appliance out of the upper-jaw oral appliance and the lower-jaw oral appliance and retains the female screw or the male screw behind the front-side retaining portion. In the oral appliance, the female screw and the male screw serve to position the lower-jaw oral appliance with respect to the upper-jaw oral appliance in a front-rear direction by rotation of the male screw threaded into the female screw on the dentition surface or behind the dentition surface.

The oral appliance can be small-sized, and the advance amount of the lower-jaw oral appliance can be easily adjusted.

According to the first aspect of the present invention, the oral appliance described above in which the front-side disposing portion is disposed on a dentition surface of the one oral appliance or behind the dentition surface is provided.

The oral appliance has excellent wearing comfortability for the user because the front-side disposing portion does not protrude frontward of the dentition.

According to the first aspect of the present invention, the rear-side retaining portion includes: a protruding portion protruding to a tongue portion side; and a rear-side facing portion that is disposed behind a dentition by the protruding portion, and retains the female screw or the male screw by facing the front-side retaining portion.

In the oral appliance, the male screw or the female screw can be disposed behind the dentition surface, and hence the screwing amount between the male screw and the female screw can be increased and the range in which the position of the lower-jaw oral appliance can be adjusted can be elongated.

According to the first aspect of the present invention, the oral appliance described above in which the front-side retaining portion retains the male screw or the female screw such that the male screw or the female screw is displaceable or turnable in a left-right direction, and the rear-side retaining portion retains the female screw or the male screw such that the female screw or the male screw is displaceable or turnable in the left-right direction is provided.

The oral appliance has excellent wearing comfortability for the user because the user wearing the oral appliance can move the lower-jaw oral appliance to the left and the right. Alternatively, the upper-jaw oral appliance or the lower-jaw oral appliance can be prevented from being damaged even when force in the left-right direction such as bruxism is applied.

According to the first aspect of the present invention, the oral appliance described above in which the rear-side retaining portion includes a turn member that turns in the left-right direction, the female screw or the male screw is retained by the rear-side retaining portion such that the female screw or the male screw is fixed to the turn member and is turnable in the left-right direction, and the male screw is allowed to turn in the left-right direction is provided.

The oral appliance has excellent wearing comfortability for the user because the user wearing the oral appliance can move the lower-jaw oral appliance to the left and the right.

According to the first aspect of the present invention, the oral appliance described above in which the turn member includes: a turn retaining portion that retains the female screw or the male screw; and a stopper that fixes a position of the turn retaining portion such that the turn retaining portion is turnable is provided.

The oral appliance has excellent wearing comfortability for the user because the user wearing the oral appliance can move the lower-jaw oral appliance to the left and the right.

According to the first aspect of the present invention, the oral appliance described above in which the front-side retaining portion includes a retaining hole formed so as to have a wide width in the left-right direction, the male screw or the female screw is retained by the front-side retaining portion so as to be displaceable in the left-right direction by being inserted through the retaining hole, and the male screw is allowed to be displaced or turn in the left-right direction is provided.

The oral appliance has excellent wearing comfortability for the user because the user wearing the oral appliance can move the lower-jaw oral appliance to the left and the right.

According to the first aspect of the present invention, the oral appliance described above in which the front-side retaining portion includes a sliding member that is displaced in the left-right direction, a position of the male screw or the female screw is fixed with respect to the sliding member, the male screw or the female screw is retained by the front-side retaining portion, and the male screw is allowed to be displaced or turn in the left-right direction is provided.

The oral appliance has excellent wearing comfortability for the user because the user wearing the oral appliance can move the lower-jaw oral appliance to the left and the right.

According to the first aspect of the present invention, the oral appliance described above in which the front-side retaining portion includes an opening portion formed so as to have a wide width in the left-right direction, the male screw or the female screw is retained by the front-side retaining portion by being inserted in the opening portion, the rear-side retaining portion includes an opening portion formed so as to have a wide width in the left-right direction, the female screw or the male screw is retained by the rear-side retaining portion by being inserted in the opening portion, and the male screw is allowed to be displaced in the left-right direction is provided.

The oral appliance has excellent wearing comfortability for the user because the user wearing the oral appliance can move the lower-jaw oral appliance to the left and the right.

According to the first aspect of the present invention, the oral appliance described above in which the rear-side retaining portion includes a pressing down portion that presses down a tongue portion of a user when a mouth is closed is provided.

The oral appliance can suppress the respiratory arrest or hypopnea during sleep in a more effective manner by preventing the constriction of the trachea of the user wearing the oral appliance because the trachea of the user is more easily opened by pressing down the tongue portion of the user wearing the oral appliance by the pressing down portion.

### 2. Oral Appliance according to Second Aspect

### [Embodiment 4]

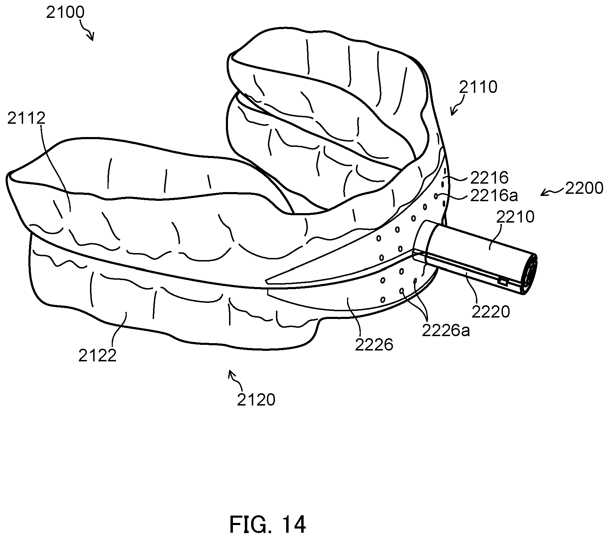

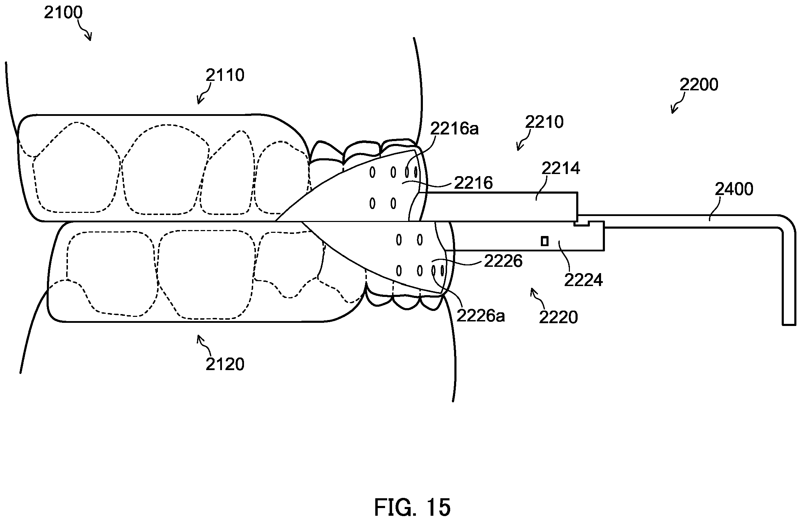

Oral appliance 2100 according to Embodiment 4 of the present invention includes one pair of an upper-jaw oral appliance and a lower-jaw oral appliance facing each other as illustrated in FIG. 14 that is a schematic perspective view illustrating the configuration of oral appliance 2100, and FIG. 15 that is a side view illustrating how the lower jaw is advanced while the oral appliance is being worn. Upper-jaw oral appliance 2110 is formed so as to be wearable on the upper jaw of the user, and lower-jaw oral appliance 2120 is formed so as to be wearable on the lower jaw of the user.

Upper-jaw oral appliance 2110 has dentition impression portion 2112 that can be worn on the dentition of the upper jaw of the user formed therein, and is formed so as to be wearable on the upper jaw of the user. Lower-jaw oral appliance 2120 has dentition impression portion 2122 that can be worn on the dentition of the lower jaw of the user formed therein, and is formed so as to be wearable on the lower jaw of the user. Lower-jaw oral appliance 2120 can be coupled to upper-jaw oral appliance 2110 disposed so as to face lower-jaw oral appliance 2120, and can be positioned frontward of upper-jaw oral appliance 2110 by lower-jaw advancing apparatus 2200 disposed on the front side thereof.

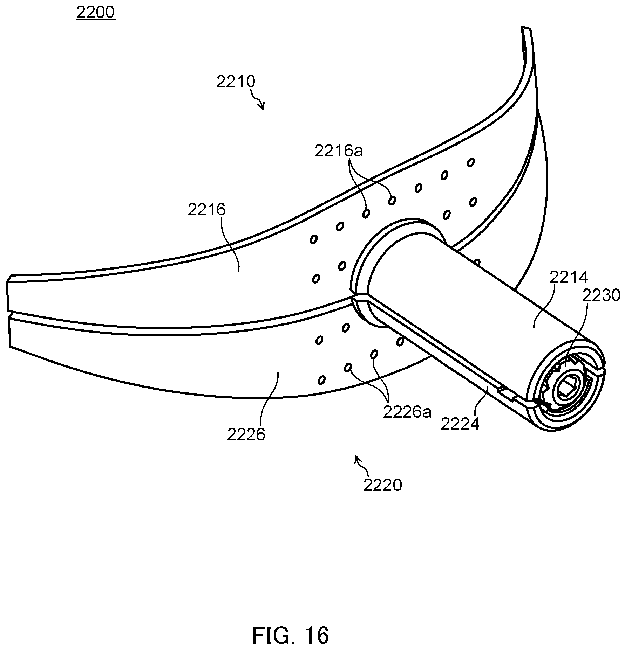

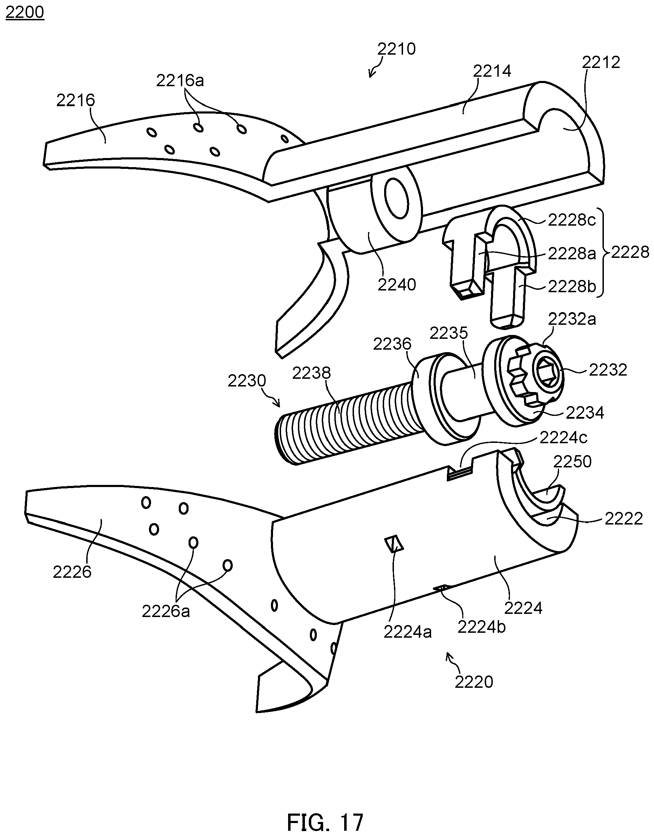

Lower-jaw advancing apparatus 2200 includes fixed member 2210 (also serving as a rear-side retaining portion) firmly fixed to upper-jaw oral appliance 2110, sliding member 2220 (also serving as a front-side retaining portion) firmly fixed to the lower-jaw oral appliance, male screw 2230 and female screw 2240 that couple fixed member 2210 and sliding member 2220 to each other and position sliding member 2220 on the front side with respect to fixed member 2210, and stopper 2250 that regulates the rotation of male screw 2230 as illustrated in FIG. 16 that is a schematic perspective view thereof and FIG. 17 that is an exploded perspective view thereof.

As illustrated in FIG. 15, lower-jaw advancing apparatus 2200 can position lower-jaw oral appliance 2120 to which sliding member 2220 is firmly fixed frontward of upper-jaw oral appliance 2110 to which fixed member 2210 is firmly fixed by adjusting the screwing amount between male screw 2230 and female screw 2240 by screwdriver 2400 and positioning sliding member 2220 with respect to fixed member 2210 in the front-rear direction.

As a result, lower-jaw advancing apparatus 2200 can limit the displacement of lower-jaw oral appliance 2120 to the rear side while moving lower-jaw oral appliance 2120 to the front side when the user wearing oral appliance 2100 interlocks the teeth while wearing upper-jaw oral appliance 2110 and lower-jaw oral appliance 2120 (hereinafter simply referred to as "at the time of dental interlocking"). As a result, oral appliance 2100 can suppress the respiratory arrest or hypopnea during sleep in a more effective manner by moving the lower jaw of the user wearing oral appliance 2100 to the front side and opening the trachea of the user in a larger manner.

Specifically, lower-jaw advancing apparatus 2200 can position lower-jaw oral appliance 2120 with respect to upper-jaw oral appliance 2110 in the front-rear direction by disposing sliding member 2220 such that sliding member 2220 faces fixed member 2210 and positioning sliding member 2220 with respect to fixed member 2210 in the front-rear direction. The position of sliding member 2220 described above can be adjusted by screwing male screw 2230 retained by sliding member 2220 together with female screw 2240 retained by fixed member 2210 and adjusting the screwing amount between male screw 2230 and female screw 2240 by rotating male screw 2230. When male screw 2230 is not rotated, stopper 2250 can suppress the change in the position of sliding member 2220 caused by the change of the screwing amount described above due to unintentional rotation of male screw 2230.

Fixed member 2210 includes substantially half-cylindrical cover portion 2214 of which surface facing sliding member 2220 serves as substantially semicircular groove portion 2212, and sliding member 2220 includes substantially half-cylindrical cover portion 2224, which is substantially the same shape as fixed member 2210 and of which surface facing fixed member 2210 serves as semicircular groove portion 2222. As a result, when fixed member 2210 and sliding member 2220 are disposed such that groove portion 2212 and groove portion 2222 face each other, cover portion 2214 and cover portion 2224 form a substantially cylindrical insertion through hole in which male screw 2230 can be inserted through groove portion 2212 and groove portion 2222.

Note that the shapes of cover portion 2214 and cover portion 2224 are not limited thereto, and may be a substantially cylindrical truncated cone shape, a substantially elliptic cylinder shape, a substantially elliptic cylindrical truncated cone shape, and the like when being disposed so as to face each other. The external shape of cover portion 2214 and the external shape of cover portion 2224 do not necessarily need to be substantially the same, and may be different shapes as long as the insertion through hole described above is formed.

Fixed member 2210 includes cover portion 2214 in which groove portion 2212 is provided in the surface facing sliding member 2220, and attachment portion 2216 for firmly fixing fixed member 2210 to upper-jaw oral appliance 2110, and retains female screw 2240.

Cover portion 2214 can be a thin-plate-like member formed in a half-cylindrical shape, for example. Cover portion 2214 may be formed in a substantially same thickness from the front-side end to the rear-side end that continues to attachment portion 2216, or the thickness of the rear-side end to which stress to the left-right direction is easily applied while the oral appliance is being worn may be formed to be thicker.

Attachment portion 2216 is a thin-plate-like member continuously extending to the left-right direction from the rear side of cover portion 2214, and is formed in a curved shape along the shape of the front-side surface of upper-jaw oral appliance 2110. Attachment portion 2216 is firmly fixed to the front-side surface of upper-jaw oral appliance 2110 by a resin material that is the same material as upper-jaw oral appliance 2110 and the like, for example. As a result, fixed member 2210 is firmly fixed to upper-jaw oral appliance 2110 on the front side thereof.

Attachment portion 2216 may include plurality of through holes 2216a for causing attachment portion 2216 to be modified and firmly fixed to the front-side surface of upper-jaw oral appliance 2110 in an easier manner. Through holes 2216a can suppress the detachment of fixed member 2210 from upper-jaw oral appliance 2110 caused by the separation of attachment portion 2216 from upper-jaw oral appliance 2110 by increasing the fixing strength of attachment portion 2216 with respect to upper-jaw oral appliance 2110 because the resin material described above can connect the front side and the rear side of attachment portion 2216 to each other through the inner portions of through holes 2216a.

Female screw 2240 is disposed in a position that is on the rear side of the insertion through hole and in the inner periphery of groove portion 2212. Female screw 2240 is integrally formed with fixed member 2210 by the same material as fixed member 2210 in this embodiment.

Female screw 2240 can be disposed on the rear end of groove portion 2212. Meanwhile, female screw 2240 is preferred to be disposed so as to be spaced from the rear end of groove portion 2212 with an interval therebetween from the viewpoint of suppressing the decrease of the wearing comfortability of the user caused when male screw 2230 that has been deeply threaded into female screw 2240 and jutted out from the rear end of the insertion through hole abuts against the tongue of the user.

Sliding member 2220 includes cover portion 2224 in which groove portion 2222 described above is provided in a surface facing fixed member 2210, attachment portion 2226 for firmly fixing sliding member 2220 to the oral appliance, and fastener 2228 that is a U-shaped mold that retains male screw 2230.

Cover portion 2224 can be a thin-plate-like member formed in a half-cylindrical shape, for example. Cover portion 2224 may be formed in a substantially same thickness from the front-side end to the rear-side end that continues to attachment portion 2226, or the thickness of the rear-side end to which stress to the left-right direction is easily applied while the oral appliance is being worn may be formed to be thicker.

Cover portion 2224 includes through holes 2224a and 2224b into which the end portions of fastener 2228 can be fitted. Cover portion 2224 may include plate portion 2224c having an edge portion on which end portion 2252 of stopper 2250 described below can be disposed.

Attachment portion 2226 is a thin-plate-like member continuously extending to the left-right direction from the rear side of cover portion 2224, and is formed in a curved shape along the shape of the front-side surface of lower-jaw oral appliance 2120. Attachment portion 2226 is firmly fixed to the front-side surface of lower-jaw oral appliance 2120 by a resin that is the same material as lower-jaw oral appliance 2120 and the like, for example. As a result, sliding member 2220 is firmly fixed to lower-jaw oral appliance 2120 on the front side thereof.

Attachment portion 2226 may include plurality of through holes 2226a for modifying attachment portion 2226 and firmly fixing attachment portion 2226 to the front-side surface of lower-jaw oral appliance 2120 in an easier manner. Through holes 2226a can suppress the detachment of sliding member 2220 from lower-jaw oral appliance 2120 caused by the separation of attachment portion 2226 from lower-jaw oral appliance 2120 by increasing the fixing strength of attachment portion 2226 with respect to lower-jaw oral appliance 2120 because the resin material described above can connect the front side and the rear side of attachment portion 2226 to each other through the inner portions of through holes 2226a.

Fastener 2228 is a U-shaped member including one pair of substantially rectangular parallelepiped fitting portions 2228a and 2228b forming the end portions of fastener 2228, and curved portion 2228c curved in a substantially semicircular shape and is coupled to fitting portions 2228a and 2228b. Fastener 2228 rotatably fixes the position of male screw 2230 to sliding member 2220 by engaging curved portion 2228c with ring-shaped narrow portion 2235 of male screw 2230 disposed along groove portion 2222 in the sliding member described below and fitting one pair of fitting portions 2228a and 2228b in corresponding through holes 2224a and 2224b. In a state in which fixed member 2210 and sliding member 2220 are not coupled to each other (male screw 2230 and female screw 2240 are not in threaded engagement), fastener 2228 can be easily removed from sliding member 2220 by inserting a pin-like member in either one of through holes 2224a and 2224b and preferably both of through holes 2224a and 2224b. As a result, fastener 2228 may easy remove male screw 2230 from the sliding member.

Male screw 2230 includes engagement portion 2232 engageable with screwdriver 2400, collar portions 2234 and 2236 disposed in two places so as to face each other, ring-shaped narrow portion 2235 disposed between collar portions 2234 and 2236, and screw portion 2238 that can be threaded into female screw 2240. Engagement portion 2232, collar portion 2234, narrow portion 2235, collar portion 2236, and screw portion 2238 are disposed in the order presented from one distal end toward the other distal end of male screw 2230.

Engagement portion 2232 is disposed on the front-side end of male screw 2230. Engagement portion 2232 only needs to have a shape that is engageable with screwdriver 2400 and enables male screw 2230 to be rotated by rotating engaged screwdriver 2400. For example, engagement portion 2232 can be a hexagon socket with a shape that can rotate male screw 2230 by inserting a distal end of hexagonal-prism-shaped screwdriver 2400 (hex key) that can be fitted in the hexagon socket therein.

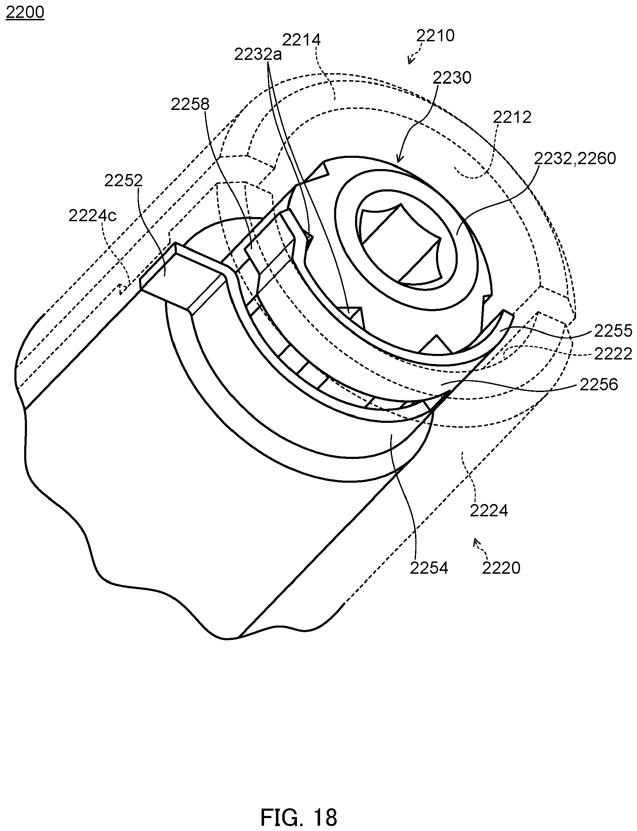

On the outer periphery of engagement portion 2232, eight recessed portions 2232a with which stopper 2250 is engageable are formed in the circumferential direction. By engaging with stopper 2250, engagement portion 2232 may also operate as rotation regulating portion 2260 for regulating the rotation of male screw 2230 (see FIG. 18).

Collar portion 2234, narrow portion 2235, and collar portion 2236 can rotatably fix the position of male screw 2230 in sliding member 2220 and remove male screw 2230 from sliding member 2220 by disposing male screw 2230 along groove portion 2222 in the sliding member and fixing fastener 2228 attached to the periphery of narrow portion 2235 to sliding member 2220.

Specifically, collar portions 2234 and 2236 are formed such that the radius of the outer periphery thereof is larger than the radius of the inner periphery of curved portion 2228c included in fastener 2228, and narrow portion 2235 is formed such that the radius of the outer periphery thereof is smaller than the radius of the inner periphery of curved portion 2228c included in fastener 2228. As a result, the movement of male screw 2230 with respect to fastener 2228 fixed to sliding member 2220 in the front-rear direction may be limited to the interval between collar portions 2234 and 2236, and the position of male screw 2230 may be rotatably fixed with respect to sliding member 2220. As described above, fastener 2228 is removable from sliding member 2220, and hence male screw 2230 is also easily removable from sliding member 2220.

Screw portion 2238 is disposed on the rear-side end of male screw 2230. Screw portion 2238 only needs to have a screw shape that can be threaded into female screw 2240. From the viewpoint of increasing the advance amount of lower-jaw oral appliance 2120 with respect to upper-jaw oral appliance 2110 by increasing the advance amount of sliding member 2220 with respect to fixed member 2210, screw portion 2238 is preferred to have a length that is half of male screw 2230 or more.

Sliding member 2220 is disposed so as to face fixed member 2210 and is coupled to fixed member 2210 by screwing screw portion 2238 of male screw 2230 retained by sliding member 2220 together with female screw 2240 retained by fixed member 2210. By adjusting the screwing amount between screw portion 2238 of male screw 2230 and female screw 2240, sliding member 2220 is positioned with respect to fixed member 2210 in the front-rear direction. As a result, lower-jaw oral appliance 2120 to which sliding member 2220 is firmly fixed can be positioned on the front side with respect to upper-jaw oral appliance 2110 retaining fixed member 2210.

At this time, by adjusting the screwing amount between screw portion 2238 of male screw 2230 and female screw 2240, as appropriate, the advance amount of lower-jaw oral appliance 2120 with respect to upper-jaw oral appliance 2110 can be adjusted. As a result, by moving lower-jaw oral appliance 2120 frontward of upper-jaw oral appliance 2110 by the amount fitted for the shape of the jaw of the user, the trachea of the user wearing oral appliance 2100 can be caused to become less likely to be constricted.

In this embodiment, as illustrated in FIG. 18 that is a transparent view of a distal end portion of lower-jaw advancing apparatus 2200, stopper 2250 is an elastic member such as a leaf spring that is disposed along the inner periphery of groove portion 2222 in sliding member 2220, and engageable with recessed portions 2232a provided on the outer periphery of rotation regulating portion 2260 (engagement portion 2232) of male screw 2230.

Stopper 2250 is intermittently engaged with recessed portions 2232a in accordance with the rotation of male screw 2230. At this time, stopper 2250 can intermittently generate collision noise and vibration by the intermittent engagement described above when male screw 2230 is rotated.

Specifically, stopper 2250 includes end portion 2252 fixed to an edge portion of groove portion 2222 included in sliding member 2220, first plate-like portion 2254, fold-over portion 2255, second plate-like portion 2256, and claw portion 2258 having a bent structure that is engageable with recessed portions 2232a.

End portion 2252 fixes the position of stopper 2250 in sliding member 2220. First plate-like portion 2254 extends from one edge portion toward the other edge portion of groove portion 2222 along the inner periphery of groove portion 2222 included in sliding member 2220, and second plate-like portion 2256 extends from the other edge portion toward one edge portion of groove portion 2222 along the inner periphery of groove portion 2222. Fold-over portion 2255 connects first plate-like portion 2254 and second plate-like portion 2256 to each other at the other end portion of groove portion 2222. Claw portion 2258 regulates unintentional rotation of male screw 2230 caused by vibration and the like while oral appliance 2100 is being worn by being engaged with recessed portions 2232a of male screw 2230.

In the lower-jaw advancing apparatuses disclosed in PTL 1 and PTL 2, it is unknown how much the screws need to be rotated in order to enable the positions of an upper-jaw oral appliance and a lower-jaw oral appliance to be adjusted to the optimal positions when the positions of the upper-jaw oral appliance and the lower-jaw oral appliance are readjusted. Therefore, a doctor and the like that adjust the advance amount of the lower-jaw oral appliance and the user and the like that adjust the advance amount of the lower-jaw oral appliance worn by the user and the like under the guidance of the doctor (hereinafter the above are also collectively simply referred to as an "adjuster") need to adjust the positions by moving the screw little by little while checking the wearing comfortability each time. Meanwhile, in this embodiment, stopper 2250 intermittently engages claw portion 2258 with recessed portions 2232a of male screw 2230 when male screw 2230 is rotated, and intermittently regulates the rotation of male screw 2230 in accordance with the rotation of male screw 2230. As a result, the adjuster that rotates male screw 2230 can perceive the rotation amount of male screw 2230 by the number of times of the collision noise or the vibration described above. Therefore, the adjuster that readjusts the positions of upper-jaw oral appliance 2110 and lower-jaw oral appliance 2120 can easily adjust upper-jaw oral appliance 2110 and lower-jaw oral appliance 2120 to suitable positions by causing the number of times of the collision noise or the vibration described above to match with the number of times in the previous adjustment.

Stopper 2250 includes first plate-like portion 2254, fold-over portion 2255, and second plate-like portion 2256, and hence the elasticity for engaging claw portion 2258 to recessed portions 2232a by the rotation of male screw 2230 can be increased, and the collision noise and the vibration generated when claw portion 2258 hits recessed portions 2232a so as to be flipped thereby at the time of the engagement described above can be increased by increasing the effective length of stopper 2250 as a leaf spring.

Stopper 2250 regulates unintentional rotation of male screw 2230 caused by vibration and the like while oral appliance 2100 is being worn by engaging claw portion 2258 with recessed portions 2232a of male screw 2230. Meanwhile, stopper 2250 allows the adjuster to intentionally rotate male screw 2230, for example, to insert screwdriver 2400 into engagement portion 2232 of male screw 2230 and rotate male screw 2230 by human strength, or to mechanically rotate male screw 2230 with use of a motor and the like. As described above, stopper 2250 can suppress unintentional positional misalignment of upper-jaw oral appliance 2110 and lower-jaw oral appliance 2120 caused when the screwing amount between male screw 2230 and female screw 2240 changes while oral appliance 2100 is being worn, for example, by regulating the rotation of male screw 2230 with respect to the axial direction.

Upper-jaw oral appliance 2110 and lower-jaw oral appliance 2120 are made of a material that is hardly damaged by a normal occlusal force of a person. Examples of the material for forming upper-jaw oral appliance 2110 and lower-jaw oral appliance 2120 may be similar to the material for forming the upper-jaw oral appliance and the lower-jaw oral appliance according to the oral appliance according to the first aspect described above, and hence overlapping descriptions are omitted.

Fixed member 2210, sliding member 2220, and male screw 2230 and female screw can be made of metal materials such as titanium or an alloy containing titanium, iron (steel including stainless steel and the like), and alloys of gold, silver, platinum, cobalt, chromium, and the like, a hard resin material (for example, polycarbonate resin) of which flexural modulus measured according to JIS T 6501 is 1000 MPa or more and 3000 MPa or less, and the like. Note that the corrosion resistance of the metal materials described above can be increased by chemical conversion coating and the like.

Fixed member 2210 can be firmly fixed to upper-jaw oral appliance 2110 by causing a photocurable material to flow into a mold for upper-jaw oral appliance 2110 in which fixed member 2210 is disposed, and curing the photocurable material by irradiating the photocurable material with an ultraviolet ray and the like. Sliding member 2220 can be firmly fixed to lower-jaw oral appliance 2120 by causing a photocurable material to flow into a mold for lower-jaw oral appliance 2120 in which sliding member 2220 is disposed, and curing the photocurable material by irradiating the photocurable material with an ultraviolet ray and the like.

Female screw 2240 may be integrally molded with fixed member 2210 by the same material as fixed member 2210. Female screw 2240 may be bonded to fixed member 2210 after being manufactured separately from fixed member 2210.

Stopper 2250 can be fixed to the edge portion of groove portion 2222 included in sliding member 2220 by welding and the like. In order to prevent modification of lower-jaw oral appliance due to heat and the like, stopper 2250 is preferred to be fixed before firmly fixing sliding member 2220 to lower-jaw oral appliance 2120.

Note that stopper 2250 may be disposed on fixed member 2210 side. However, from the viewpoint of causing stopper 2250 and male screw to be easily engaged with each other even when the sliding member is advanced, stopper 2250 is preferred to be disposed on sliding member 2220.

The shape or the structure of stopper 2250 is not particularly limited. For example, stopper 2250 may be a coil spring and the like disposed along the inner periphery of groove portion 2212 in fixed member 2210 or groove portion 2222 in sliding member 2220, and a member and the like having a shape that is engageable with male screw recessed portions 2232a may be provided on the distal end of the coil spring.

Recessed portions 2232a do not necessarily need to be provided on the outer periphery of engagement portion 2232 of male screw 2230, and may be provided on the outer periphery between engagement portion 2232 and collar portion 2234 or between collar portion 2236 and screw portion 2238. However, from the viewpoint of causing the collision noise or the vibration described above to be easily sensed by the adjuster, recessed portions 2232a are preferred to be provided on a side closer to the front-side end of male screw 2230, and is more preferred to be provided on the outer periphery of engagement portion 2232.

The number of recessed portions 2232a is not particularly limited, and may be only one, or may be two, three, four, six, eight, and the like. When plurality of recessed portions 2232a are provided, plurality of recessed portions 2232a are preferred to be provided in positions that are rotationally symmetric about the rotation center of male screw 2230.

### [Embodiment 5]

An oral appliance according to Embodiment 5 of the present invention has a configuration similar to that of oral appliance 2100 according to Embodiment 4 besides the configuration of the stopper being different. Therefore, stopper 2270 having a different configuration from that of Embodiment 4 is described and the description of other common configurations is omitted below.

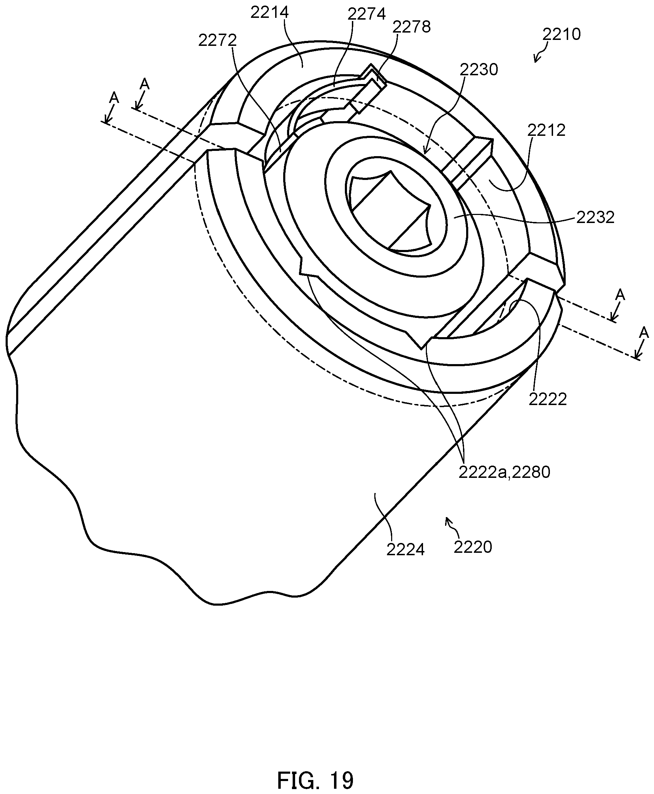

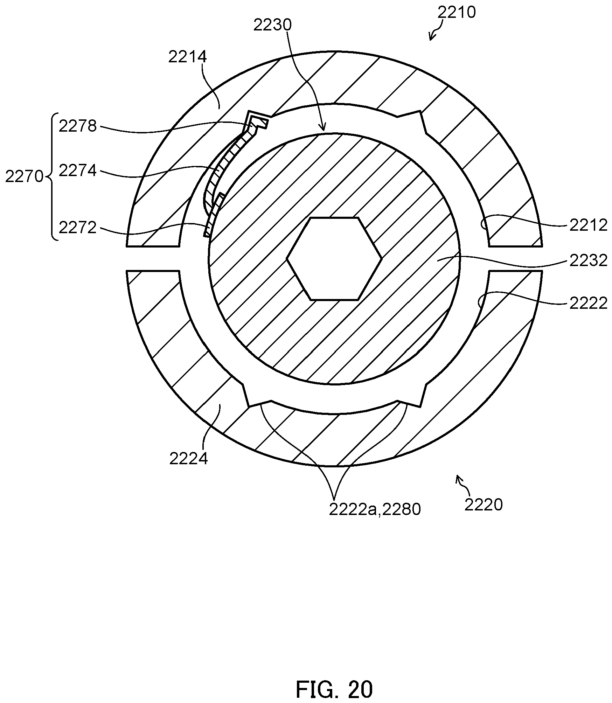

In this embodiment, as illustrated in FIG. 19 that is a perspective view of a distal end portion of lower-jaw advancing apparatus 2200 and FIG. 20 that is a cross-sectional view taken along line A-A in FIG. 19, stopper 2270 is an elastic member such as a leaf spring that is retained by male screw 2230, disposed on the outer periphery of male screw 2230, and engageable with recessed portions 2222a (also serving as rotation regulating portions 2280) provided in the inner periphery of groove portion 2212 in fixed member 2210 and groove portion 2222 in sliding member 2220.

Also in this embodiment, stopper 2270 intermittently engages with recessed portions 2222a in accordance with the rotation of male screw 2230. At this time, stopper 2270 can intermittently generate collision noise and vibration by the intermittent engagement described above when male screw 2230 is rotated.

Specifically, stopper 2270 includes end portion 2272 fixed to the outer periphery of engagement portion 2232 of male screw 2230, plate-like portion 2274 provided so as to be bent toward the inner periphery of the insertion through hole, and claw portion 2278 having a bent structure that is engageable with recessed portions 2222a.

End portion 2272 fixes the position of stopper 2270 to the outer periphery of engagement portion 2232 included in male screw 2230. Plate-like portion 2274 extends from male screw 2230 toward the inner periphery of the insertion through hole while being bent along the inner periphery of the insertion through hole. Claw portion 2278 regulates unintentional rotation of male screw 2230 caused by vibration and the like while the oral appliance is being worn by being engaged with recessed portions 2222a in fixed member 2210 and sliding member 2220.

In stopper 2270, claw portion 2278 intermittently engages with recessed portions 2222a in fixed member 2210 and sliding member 2220 when male screw 2230 is rotated, and the rotation of male screw 2230 is intermittently regulated in accordance with the rotation of male screw 2230. As a result, the adjuster that rotates male screw 2230 can perceive the rotation amount of male screw 2230 by the number of times of the collision noise or the vibration described above. Therefore, the adjuster that readjusts the positions of upper-jaw oral appliance 2110 and lower-jaw oral appliance 2120 can easily adjust upper-jaw oral appliance 2110 and lower-jaw oral appliance 2120 to suitable positions by causing the number of times of the collision noise or the vibration described above to match with the number of times in the previous adjustment.

By adjusting the length of plate-like portion 2274 and increasing the effective length of stopper 2270 as a leaf spring, stopper 2270 can increase the elasticity thereof for engaging claw portion 2278 with recessed portions 2222a by the rotation of male screw 2230, and can increase the collision noise and the vibration generated when claw portion 2278 hits recessed portions 2222a so as to be flipped thereby at the time of the engagement described above.

Stopper 2270 regulates unintentional rotation of male screw 2230 caused by vibration and the like while the oral appliance is being worn by engaging claw portion 2278 with groove portion 2212 in fixed member 2210 or groove portion 2222 in sliding member 2220. Meanwhile, stopper 2270 allows the adjuster to intentionally rotate male screw 2230, for example, to insert screwdriver 2400 into rotation regulating portion 2280 of male screw 2230 and rotate male screw 2230 by human strength, or to mechanically rotate male screw 2230 with use of a motor and the like. As described above, stopper 2270 can suppress unintentional positional misalignment of upper-jaw oral appliance 2110 and lower-jaw oral appliance 2120 caused when the screwing amount between male screw 2230 and female screw 2240 changes while the oral appliance is being worn, for example, by regulating the rotation of male screw 2230 with respect to the axial direction.

Note that it is possible to form recessed portions 2222a only in fixed member 2210, or only in sliding member 2220.

The number of recessed portions 2222a is not particularly limited, and may be only one, or may be two, three, four, six, eight, and the like. When plurality of recessed portions 2222a are provided, plurality of recessed portions 2222a are preferred to be provided in positions that are rotationally symmetric about the rotation center of male screw 2230.

Stopper 2270 does not necessarily need to be provided on the outer periphery of engagement portion 2232 of male screw 2230, and may be provided on the outer periphery between engagement portion 2232 and collar portion 2234 or between collar portion 2236 and screw portion 2238. However, from the viewpoint of causing the collision noise or the vibration described above to be easily sensed by the adjuster, stopper 2270 is preferred to be provided on the side close to the front-side end of male screw 2230, and is more preferred to be provided on the outer periphery of engagement portion 2232.

### [Embodiment 6]

An oral appliance according to Embodiment 6 of the present invention has a configuration similar to that of oral appliance 2100 according to Embodiment 4 besides the configuration of the lower-jaw advancing apparatus being different. Therefore, lower-jaw advancing apparatus 2300 having a different configuration from that of Embodiment 4 is described and the description of common configurations is omitted below.

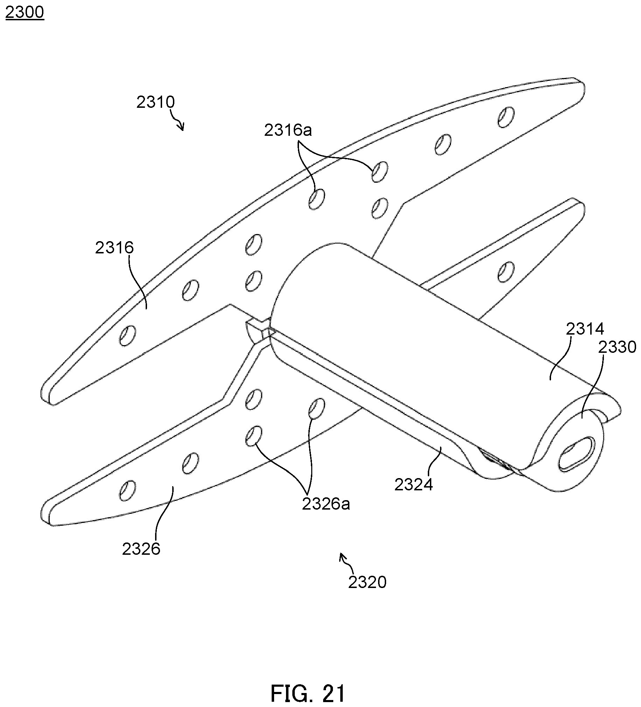

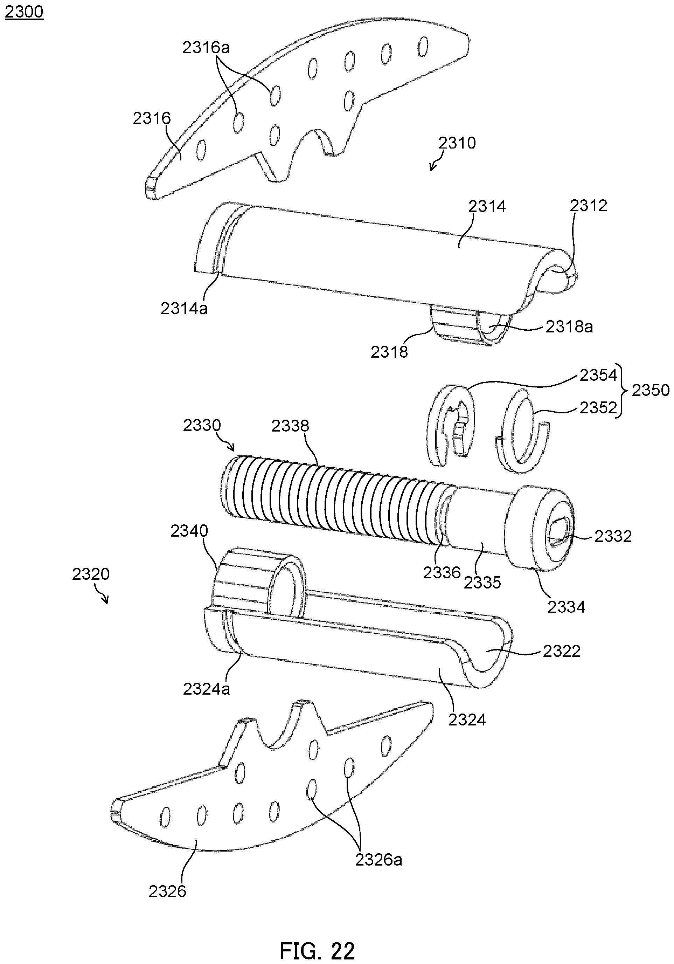

Lower-jaw advancing apparatus 2300 includes fixed member 2310 (also serving as a rear-side retaining portion) firmly fixed to lower-jaw oral appliance 2120, sliding member 2320 (also serving as a front-side retaining portion) firmly fixed to upper-jaw oral appliance 2110, male screw 2330 and female screw 2340 that couple fixed member 2310 and sliding member 2320 to each other and position sliding member 2320 on the front side with respect to fixed member 2310, and stopper 2350 that regulates the rotation of male screw 2330 as illustrated in FIG. 21 that is a schematic perspective view thereof and FIG. 22 that is an exploded perspective view thereof.

As with Embodiment 4, lower-jaw advancing apparatus 2300 can position lower-jaw oral appliance 2120 to which fixed member 2310 is firmly fixed frontward of upper-jaw oral appliance 2110 to which sliding member 2320 is firmly fixed by adjusting the screwing amount between male screw 2330 and female screw 2340 by a screwdriver and positioning sliding member 2320 with respect to fixed member 2310 in the front-rear direction.

Fixed member 2310 includes substantially half-cylindrical cover portion 2314 of which surface facing sliding member 2320 serves as substantially semicircular groove portion 2312, and sliding member 2320 includes substantially half-cylindrical cover portion 2324 of which surface facing fixed member 2310 serves as semicircular groove portion 2322. As a result, when fixed member 2310 and sliding member 2320 are disposed such that groove portion 2312 and groove portion 2322 face each other, cover portion 2314 and cover portion 2324 form a substantially cylindrical insertion through hole in which male screw 2330 can be inserted through groove portion 2312 and groove portion 2322. Note that, in this embodiment, sliding member 2320 is formed to have a shorter length than fixed member 2310 in the front-rear direction, and fixed member 2310 and sliding member 2320 form a substantially cylindrical member having a cutout portion at which male screw 2330 is exposed in a distal end portion thereof when fixed member 2310 and sliding member 2320 are disposed so as to face each other.

Note that the shapes of cover portion 2314 and cover portion 2324 are not limited to the above, and may have a substantially cylindrical truncated cone shape, a substantially elliptic cylinder shape, a substantially elliptic cylindrical truncated cone shape, and the like when cover portion 2314 and cover portion 2324 are disposed so as to face each other. Cover portion 2314 and cover portion 2324 may be members, which have the substantially same length in the front-rear direction and in which male screw 2330 is not exposed at the distal end portion thereof when cover portion 2314 and cover portion 2324 are disposed so as to face each other.

Fixed member 2310 includes cover portion 2314 in which groove portion 2312 is provided in a surface facing sliding member 2320, and attachment portion 2316 for firmly fixing fixed member 2310 to lower-jaw oral appliance 2120.

Cover portion 2314 can be a thin-plate-like member formed in a half-cylindrical shape, for example. Cover portion 2314 may be formed in a substantially same thickness from the front-side end to the rear-side end that continues to attachment portion 2316, or the thickness of the rear-side end to which stress to the left-right direction is easily applied while the oral appliance is being worn may be formed to be thicker. In this embodiment, cover portion 2314 may have groove portion 2314a into which attachment portion 2316 is fitted in the outer periphery on the rear side.

Attachment portion 2316 is a flat-plate-like member extending in the left-right direction and installed in cover portion 2314 on the rear side thereof by being fitted in groove portion 2314a, and is firmly fixed to the front-side surface of lower-jaw oral appliance 2120 by being embedded in the inner portion of the front-side surface of lower-jaw oral appliance 2120. As a result, fixed member 2310 is firmly fixed to lower-jaw oral appliance 2120 on the front side thereof.

Attachment portion 2316 may include plurality of through holes 2316a for firmly fixing attachment portion 2316 to the front-side surface of lower-jaw oral appliance 2120 in an easier manner. Through holes 2316a can suppress the detachment of fixed member 2310 from lower-jaw oral appliance 2120 caused by the separation of attachment portion 2316 from lower-jaw oral appliance 2120 by increasing the fixing strength of attachment portion 2316 with respect to lower-jaw oral appliance 2120 because the resin material described above can connect the front side and the rear side of attachment portion 2316 to each other through the inner portions of through holes 2316a.

Sliding member 2320 includes cover portion 2324 in which groove portion 2322 is provided in the surface facing fixed member 2310, attachment portion 2326 for firmly fixing sliding member 2320 to upper-jaw oral appliance 2110, and fastener 2328 for retaining the male screw.

Cover portion 2324 can be a thin-plate-like member formed in a half-cylindrical shape, for example. Cover portion 2324 may be formed in a substantially same thickness from the front-side end to the rear-side end that continues to attachment portion 2326, or the thickness of the rear-side end to which stress to the left-right direction is easily applied while the oral appliance is being worn may be formed to be thicker. In this embodiment, cover portion 2324 has groove portion 2324a into which attachment portion 2326 is fitted in the outer periphery on the rear side.

Attachment portion 2326 is a flat-plate-like member extending in the left-right direction and installed in cover portion 2324 on the rear side thereof by being fitted in groove portion 2324a, and is firmly fixed to the front-side surface of upper-jaw oral appliance 2110 by being embedded in the inner portion of the front-side surface of upper-jaw oral appliance 2110. As a result, sliding member 2320 is firmly fixed to upper-jaw oral appliance 2110 on the front side thereof.

Attachment portion 2326 may include plurality of through holes 2326a for causing attachment portion 2326 to be firmly fixed to the front-side surface of upper-jaw oral appliance 2110 in easier manner. Through holes 2326a can suppress the detachment of sliding member 2320 from upper-jaw oral appliance 2110 caused by the separation of attachment portion 2326 from upper-jaw oral appliance 2110 by increasing the fixing strength of attachment portion 2326 with respect to upper-jaw oral appliance 2110 because the resin material described above can connect the front side and the rear side of attachment portion 2326 to each other through the inner portions of through holes 2326a.

Fastener 2328 is a hollow cylindrical member including insertion through hole 2328a that retains male screw 2330 by inserting male screw 2330 therethrough. Insertion through hole 2328a has a through hole with a size of which outer diameter is larger than cylindrical portion 2335 of male screw 2330 described below and smaller than the outer diameters of collar portion 2334 of male screw 2330 and locking member 2354 described below, and retains male screw 2330 in sliding member 2320 by rotatably fixing the position of male screw 2330 inserted through the through hole described above to fixed member 2310.

Male screw 2330 includes engagement portion 2332 engageable with a screwdriver, collar portion 2334, cylindrical portion 2335, ring-shaped narrow portion 2336 formed in a position that is behind fastener 2328 when male screw 2330 is firmly fixed to fixed member 2310, and screw portion 2338 that can be threaded into female screw 2340. Engagement portion 2332, collar portion 2334, narrow portion 2336, and screw portion 2338 are disposed in the order presented from one distal end toward the other distal end of male screw 2330.

Engagement portion 2332 is disposed on the front-side end of male screw 2330 or between the front-side end and fastener 2328. Engagement portion 2332 only needs to have a shape that is engageable with a screwdriver and enables rotate male screw 2330 to be rotated by rotating the engaged screwdriver. For example, engagement portion 2332 can be an elliptic-cylindrical hole, and can have a shape that rotates male screw 2330 by inserting the distal end of an elliptic-cylindrical screwdriver that can be fitted thereinto.

Collar portion 2334 is formed such that the radius of the outer periphery thereof is larger than the radius of the inner periphery of insertion through hole 2328a included in fastener 2328. Cylindrical portion 2335 is a portion disposed on the inner portion of insertion through hole 2328a when male screw 2330 is inserted through insertion through hole 2328a included in fastener 2328, and is formed such that the radius of the outer periphery thereof is smaller than the radius of the inner periphery of insertion through hole 2328a included in fastener 2328. Narrow portion 2336 is formed such that the radius of the outer periphery thereof is smaller than the radius of the outer periphery of cylindrical portion 2335, and in a size in which locking member 2354 can be fitted.

Screw portion 2338 is disposed on the rear-side portion of male screw 2330. Screw portion 2338 only needs to have a screw shape that can be threaded into female screw 2340. From the viewpoint of increasing the advance amount of lower-jaw oral appliance 2120 with respect to upper-jaw oral appliance 2110 by increasing the advance amount of sliding member 2320 with respect to fixed member 2310, screw portion 2338 is preferred to have a length that is half of male screw 2330 or more.

Female screw 2340 is disposed in a position that is on the rear side of the insertion through hole described above formed by groove portion 2312 and groove portion 2322 on the inner periphery of groove portion 2322 included in sliding member 2320. Female screw 2340 is integrally formed with sliding member 2320 by the same material as sliding member 2320 in this embodiment.

Female screw 2340 can be disposed on the rear end of groove portion 2322. Meanwhile, from the viewpoint of suppressing the decrease of the wearing comfortability of the user caused when male screw 2330 that has been deeply threaded into female screw 2340 and jutted out from the rear end of the insertion through hole abuts against the tongue of the user, female screw 2340 is preferred to be disposed so as to be spaced from the rear end of groove portion 2322 with an interval therebetween.

Sliding member 2320 is disposed so as to face fixed member 2310 and is coupled to fixed member 2310 by screwing screw portion 2338 of male screw 2330 retained by sliding member 2320 together with female screw 2340 retained by fixed member 2310. By adjusting the screwing amount between screw portion 2338 of male screw 2330 and female screw 2340, sliding member 2320 is positioned with respect to fixed member 2310 in the front-rear direction. As a result, lower-jaw oral appliance 2120 retaining fixed member 2310 can be positioned on the front side with respect to upper-jaw oral appliance 2110 to which sliding member 2320 is firmly fixed.

At this time, the advance amount of lower-jaw oral appliance 2120 with respect to upper-jaw oral appliance 2110 can be adjusted by adjusting the screwing amount between screw portion 2338 of male screw 2330 and female screw 2340, as appropriate. As a result, by moving lower-jaw oral appliance 2120 frontward of upper-jaw oral appliance 2110 by the amount fitted for the shape of the jaw of the user, the trachea of the user wearing oral appliance 2100 can be caused to become less likely to be constricted.

In this embodiment, as illustrated in FIG. 22, stopper 2350 includes compression coil spring 2352 that is a coiled elastic member through which cylindrical portion 2335 of male screw 2330 is inserted, and substantially-U-shaped locking member 2354 fitted onto narrow portion 2336 of male screw 2330. Both of compression coil spring 2352 and locking member 2354 are formed such that the radius of the outer periphery thereof is larger than the radius of the inner periphery of insertion through hole 2328a included in fastener 2328. Stopper 2350 is placed in a state in which cylindrical portion 2335 of male screw 2330 is inserted through compression coil spring 2352 and locking member 2354 is fitted on narrow portion 2336 of male screw 2330 when male screw 2330 is inserted through fastener 2328. Therefore, when male screw 2330 is inserted through fastener 2328, collar portion 2334 of male screw 2330, compression coil spring 2352, fastener 2328 (cylindrical portion 2335 of male screw 2330), and locking member 2354 (narrow portion 2336 of male screw 2330) are disposed in the order presented from one distal end toward the other distal end of male screw 2330. At this time, neither of compression coil spring 2352 and locking member 2354 can pass through insertion through hole 2328a included in fastener 2328, and hence the position of male screw 2330 is rotatably fixed with respect to sliding member 2320 by limiting the movement of male screw 2330 with respect to fastener 2328 fixed to sliding member 2320 in the front-rear direction.

In stopper 2350, when screw portion 2338 of male screw 2330 and female screw 2340 are in threaded engagement, compression coil spring 2352 biases collar portion 2334 of male screw 2330 to the front side (the direction away from female screw 2340). Meanwhile, the displacement of male screw 2330 to the front side (the direction away from female screw 2340) is regulated by causing locking member 2354 to abut against fastener 2328. By the biasing and the regulation of the displacement described above, stopper 2350 brings male screw 2330 into contact with fastener 2328 by pressurizing, and regulates unintentional rotation of male screw 2330 caused by vibration and the like while oral appliance 2100 is being worn. Meanwhile, stopper 2350 allows the adjuster to intentionally rotate male screw 2330, for example, to insert a screwdriver into engagement portion 2332 of male screw 2330 and rotate male screw 2330 with human strength, or to mechanically rotate male screw 2330 with use of a motor and the like. As described above, stopper 2350 can suppress unintentional positional misalignment of upper-jaw oral appliance 2110 and lower-jaw oral appliance 2120 caused when the screwing amount between male screw 2330 and female screw 2340 changes while oral appliance 2100 is being worn, for example, by regulating the rotation of male screw 2330 with respect to the axial direction.

Note that, also in this embodiment, as with Embodiment 4 and Embodiment 5, fastener 2328 may be a U-shaped mold member including one pair of substantially rectangular parallelepiped fitting portions fitted into through holes formed in cover portion 2324 of sliding member 2320, and cover portion 2324 of sliding member 2320 may include through holes in which the fitting portions of fastener 2328 described above can be fitted. At this time, in a state in which fixed member 2310 and sliding member 2320 are not coupled to each other (male screw 2330 and female screw 2340 are not in threaded engagement), fastener 2328 can be easily removed from sliding member 2320 by inserting a pin-like member in either one of the through holes included in cover portion 2314 and preferably both of the through holes. As a result, fastener 2328 may easily remove male screw 2330 from sliding member 2320.

Also in this embodiment, instead of fitting narrow portion 2336 of male screw 2330 into locking member 2354, a collar portion having a larger outer diameter than the radius of the inner periphery of insertion through hole 2328a included in fastener 2328 may be formed on cylindrical portion 2335 of male screw 2330 on the rear side thereof, and the displacement of male screw 2330 to the front side (the direction away from female screw 2340) may be regulated by causing the collar portion on the rear side described above to abut against fastener 2328.

Compression coil spring 2352 only needs to bias collar portion 2334 of male screw 2330 to the front side when the screwing amount between male screw 2330 and female screw 2340 becomes large (when lower-jaw oral appliance 2120 moves frontward of upper-jaw oral appliance 2110), and may be an elastic body such as rubber.

### [Embodiment 7]

An oral appliance according to Embodiment 7 of the present invention has a similar configuration as the oral appliance according to Embodiment 6 besides some configurations of lower-jaw advancing apparatus being different. Therefore, out of the configuration of lower-jaw advancing apparatus, the configuration different from that in Embodiment 6 is described and the description of common configurations is omitted below.

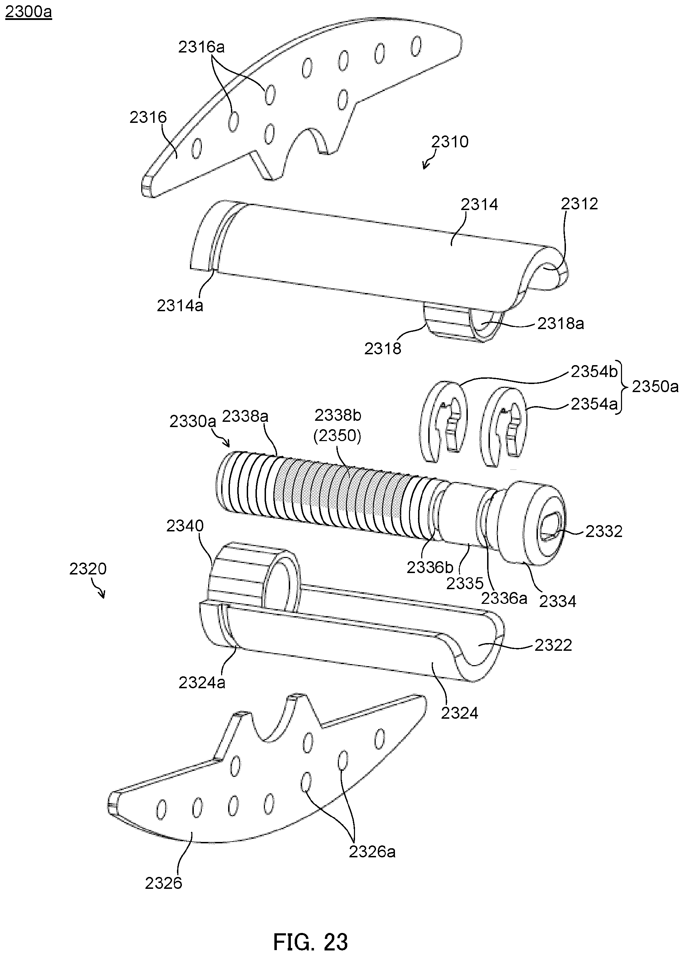

In lower-jaw advancing apparatus 2300a, male screw 2330a includes one pair of narrow portions 2336a and 2336b, and stopper 2350a includes one pair of locking members 2354a and locking member 2354b as illustrated in FIG. 23 that is a schematic exploded perspective view thereof. Both of the locking member 2354a and locking member 2354b are formed such that the radius of the outer periphery thereof is larger than the radius of the inner periphery of insertion through hole 2328a included in fastener 2328. When male screw 2330a is inserted through fastener 2328, stopper 2350a is placed in a state in which locking member 2354a is fitted on narrow portion 2336a of male screw 2330a and locking member 2354b is fitted on narrow portion 2336b of male screw 2330a. Therefore, when male screw 2330a is inserted through fastener 2328, collar portion 2334 of male screw 2330a, locking member 2354a (narrow portion 2336a of male screw 2330a), fastener 2328 (cylindrical portion 2335 of male screw 2330a), and locking member 2354b (narrow portion 2336b of male screw 2330a) are disposed in the order presented from one distal end toward the other distal end of male screw 2330. At this time, neither of locking member 2354a and locking member 2354b can pass through insertion through hole 2328a included in fastener 2328, and hence the movement of male screw 2330a with respect to fastener 2328 fixed to sliding member 2320 in the front-rear direction is limited, and the position of male screw 2330a is rotatably fixed with respect to sliding member 2320.

Stopper 2350a includes elastic coating 2338b adhering to the surface of screw portion 2338a of male screw 2330a. Elastic coating 2338b increases the frictional force between screw portion 2338a of male screw 2330a and female screw 2340, increases the torque for screwing male screw 2330a with respect to female screw 2340, and regulates unintentional rotation of male screw 2330a caused by vibration and the like while the oral appliance is being worn. Meanwhile, elastic coating 2338b allows the adjuster to intentionally rotate male screw 2330a, for example, to insert a screwdriver into engagement portion 2332 of male screw 2330a and rotate male screw 2330a with human strength, or to mechanically rotate male screw 2330a with use of a motor and the like. As described above, elastic coating 2338b serving as a stopper can suppress unintentional positional misalignment of upper-jaw oral appliance 2110 and lower-jaw oral appliance 2120 caused when the screwing amount between male screw 2330a and female screw 2340 changes while the oral appliance is being worn, for example, by regulating the rotation of male screw 2330a with respect to the axial direction.

Elastic coating 2338b may be applied on the entire surface of screw portion 2338a or may be applied on only a part thereof. When elastic coating 2338b is applied on only a part of screw portion 2338a, from the viewpoint of efficiently increasing the frictional force between screw portion 2338a and female screw 2340, elastic coating 2338b is preferred to be applied on a surface facing the same direction with respect to the axis of rotation of screw portion 2338a.

Examples of elastic coating 2338b include urethane-based resin and the like.

Note that, also in this embodiment, as with Embodiment 6, a compression coil spring may be disposed instead of locking member 2354a, and the biasing by the compression coil spring and the regulation of the rotation of male screw 2330a with respect to the axial direction by the regulation of the displacement by the locking member 2354b may be simultaneously performed.

### [Embodiment 8]

The oral appliance according to Embodiment 5 of the present invention has a configuration similar to that of the oral appliance according to Embodiment 6 besides some configurations of lower-jaw advancing apparatus being different. Therefore, out of the configuration of lower-jaw advancing apparatus, the configuration different from that in Embodiment 6 is described, and the description of common configurations is omitted below. Note that, in this embodiment, fixed member 2310 is firmly fixed to upper-jaw oral appliance 2110, and sliding member 2320 is firmly fixed to lower-jaw oral appliance 2120.

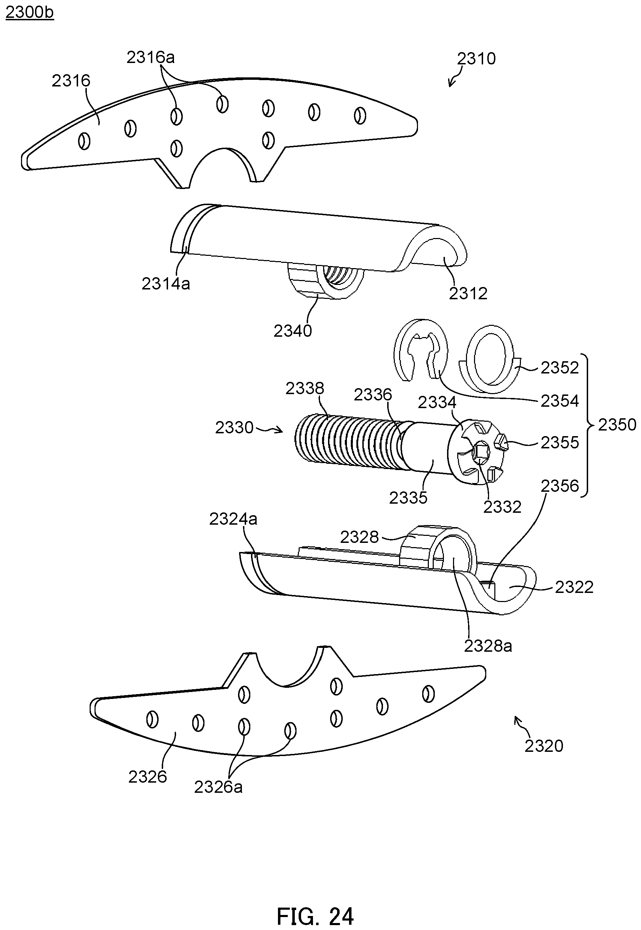

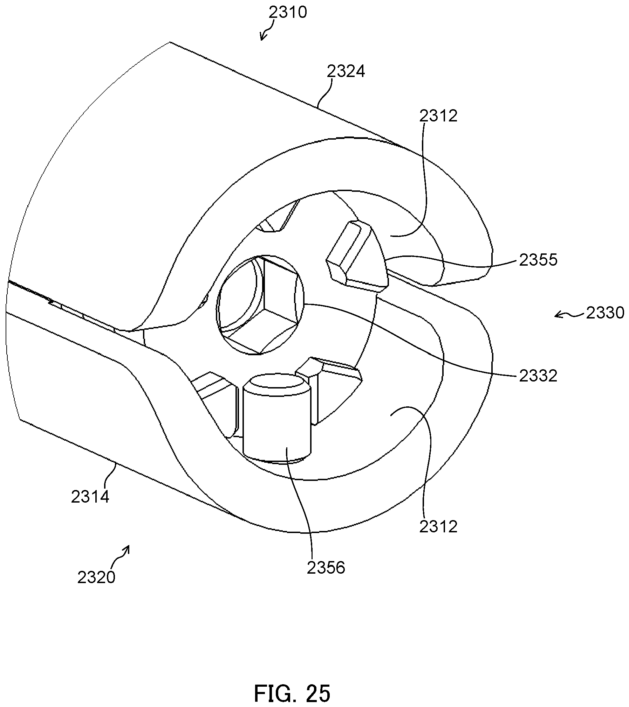

In lower-jaw advancing apparatus 2300b, sliding member 2320 includes fastener 2328 that is a hollow cylindrical member including insertion through hole 2328a that retains male screw 2330 that is inserted therethrough, and fixed member 2310 includes female screw 2340 as illustrated in FIG. 24 that is a schematic exploded perspective view thereof and FIG. 25 that is an enlarged a perspective view of a distal end portion thereof.

In this embodiment, as illustrated in FIG. 22, stopper 2350 includes compression coil spring 2352 that is a coiled elastic member through which cylindrical portion 2335 of male screw 2330 is inserted, substantially-U-shaped locking member 2354 that is fitted on narrow portion 2336 of male screw 2330, locking pieces 2355 formed so as to protrude from collar portion 2334 of male screw 2330, and protruding locking portion 2356 formed so as to protrude to a position to be engaged with locking pieces 2355 of male screw 2330 out of the inner periphery surface of fixed member 2310 or sliding member 2320.

Locking pieces 2355 are members circumferentially disposed on the front-side surface of collar portion 2334 of male screw 2330 along the outer periphery of the front-side surface, and protruding to the front side from the front-side surface described above. Locking pieces 2355 are disposed such that gaps are formed. The gaps enable protruding locking portion 2356 protruding from the inner periphery surface of fixed member 2310 or sliding member 2320 to be disposed between plurality of locking pieces 2355 and enable the side surfaces of prism-shaped locking pieces 2355 to come into contact with the side surface of protruding locking portion 2356. Note that, in FIG. 24, locking pieces 2355 each have a substantially triangular prism shape, but may have other prism shapes such as a quadrangular prism shape or a cylindrical shape as long as the gaps described above are formed. Alternatively, locking pieces 2355 may have concentric circular pillar shapes having the gaps described above. In FIG. 24, five locking pieces 2355 are formed on the front-side surface described above, but the number of locking pieces 2355 is not limited and may be one or plurality. Note that the height of each of locking pieces 2355 protruding from the front-side surface described above is a height that enables the below. That is, the side surface of locking piece 2355 can normally come into contact with the side surfaces of protruding locking portions 2356. However, when compression coil spring 2352 is compressed and male screw 2330 moves to the rear side, the side surfaces of locking pieces 2355 also move to a position that does not come into contact with the side surface of protruding locking portion 2356.

Protruding locking portion 2356 is a member disposed so as to protrude from the inner periphery surface of fixed member 2310 or sliding member 2320. Protruding locking portion 2356 is fitted into the gaps described above formed by locking pieces 2355, and normally regulates the rotation of male screw 2330 with respect to the axial direction by coming into contact with the side surfaces of locking pieces 2355. Meanwhile, protruding locking portion 2356 does not come into contact with the side surfaces of locking pieces 2355 and does not regulate the rotation of male screw 2330 when male screw 2330 is pressed by inserting a screwdriver into engagement portion 2332 of male screw 2330a, compression coil spring 2352 is compressed, and male screw 2330 is moved to the rear side. As described above, compression coil spring 2352 operates as a contact switching portion that switches between a state in which locking pieces 2355 and protruding locking portion 2356 are engaged with each other and the rotation of male screw 2330 is regulated, and a state in which locking pieces 2355 and protruding locking portion 2356 are not engaged with each other and the rotation of male screw 2330 is not regulated.

Note that, also in this embodiment, as with Embodiment 6, when screw portion 2338 of male screw 2330 and female screw 2340 are in threaded engagement, compression coil spring 2352 biases collar portion 2334 of male screw 2330 to the front side (the direction away from female screw 2340), and regulates the displacement of male screw 2330 to the front side (the direction away from female screw 2340) by causing locking member 2354 to abut against fastener 2328. By the biasing and the regulation of the displacement described above, stopper 2350 regulates unintentional rotation of male screw 2330 caused by vibration and the like while oral appliance 2100 is being worn also by bringing male screw 2330 into contact with fastener 2328 by pressurizing.

Meanwhile, stopper 2350 allows the adjuster to intentionally rotate male screw 2330, for example, to insert a screwdriver into engagement portion 2332 of male screw 2330 and rotate male screw 2330 with human strength, or to mechanically rotate male screw 2330 with use of a motor and the like. As described above, stopper 2350 can suppress unintentional positional misalignment of upper-jaw oral appliance 2110 and lower-jaw oral appliance 2120 caused when the screwing amount between male screw 2330 and female screw 2340 changes while oral appliance 2100 is being worn, for example, by regulating the rotation of male screw 2330 with respect to the axial direction.

Note that, also in this embodiment, as with Embodiment 7, unintentional rotation of male screw 2330a caused by vibration and the like while the oral appliance is being worn may be regulated by causing elastic coating 2338b to adhere to the surface of screw portion 2338a of male screw 2330a, increasing the frictional force between screw portion 2338a of male screw 2330a and female screw 2340, and increasing the torque for screwing male screw 2330a with respect to female screw 2340.

### [Modified Examples of Embodiment 4 to Embodiment 8]

Note that each of Embodiment 4 to Embodiment 8 describes one example of a second aspect of the present invention, and it goes without saying that the second aspect of the present invention is not limited to Embodiment 4 to Embodiment 8, and other various embodiments are also possible within the scope of the idea of the present invention.

For example, in Embodiment 4 to Embodiment 8, the female screw is integrally molded with the fixed member or is bonded to the fixed member, but the female screw may be removably retained by fixed member. Meanwhile, in Embodiment 4 to Embodiment 8, the male screw is removably retained by sliding member, but the male screw may be firmly fixed to the sliding member.

In Embodiment 4 to Embodiment 8, the male screw or the female screw may have partially different torques for screwing the male screw or the female screw.

For example, the screw portion of the male screw may have two types of outer diameters in which the outer diameter on the distal end portion side on the rear side is larger than the outer diameter on the collar portion side, or may have a shape in which the outer diameter continuously increases from the collar portion side to the distal end portion side on the rear side. Alternatively, in the screw portion of the male screw, the outer diameter of the distal end portion on the rear side may be enlarged so as to extend to the outer side by providing a gap portion or a slit from the distal end on the rear side in collar portion direction. The outer diameter of the distal end portion on the rear side can be increased by opening the distal end portion on the rear side to the outer side by changing the width of the slit in the depth direction such that the width of the distal end portion on the rear side increases. Alternatively, the female screw may have two types of inner diameters in which the inner diameter on the front side is smaller than the inner diameter on the rear side, or may have a shape in which the inner diameter continuously decreases from the rear side to the front side.

Alternatively, the screw portion of the male screw or the female screw may have a small-pitch portion in which the pitch of the screw partially decreases. The small-pitch portion may have a pitch with a fixed width that is smaller than other regions, or may have a pitch that continuously decreases toward the front side or the rear side.

In Embodiment 4 to Embodiment 8, the fixed member is firmly fixed to upper-jaw oral appliance, and the sliding member is firmly fixed to the lower-jaw oral appliance, but the sliding member retaining the male screw may be firmly fixed to the upper-jaw oral appliance, and the fixed member retaining the female screw may be firmly fixed to the lower-jaw oral appliance. At this time, the directions of the male screw and the female screw are opposite to those in Embodiment 4 to Embodiment 8, and the engagement portion of the male screw is disposed so as to face the rear side of the oral appliance.

In Embodiment 4 to Embodiment 8, the lower-jaw advancing apparatus includes either one of the stopper retained by the fixed member or the sliding member or the stopper retained by the male screw, but may include both of the stopper retained by the fixed member or the sliding member and the stopper retained by the male screw such that the positions thereof are shifted in the front-rear direction.

In Embodiment 4 to Embodiment 8, the lower-jaw advancing apparatus is disposed on the front side of the upper-jaw oral appliance and the lower-jaw oral appliance, but the lower-jaw advancing apparatus may be disposed between the dentitions of the upper-jaw oral appliance and the lower-jaw oral appliance or may be disposed on the rear side of the upper-jaw oral appliance and the lower-jaw oral appliance.

In Embodiment 4 to Embodiment 8, the dentition impression portions that can be worn on the dentition are formed in the upper-jaw oral appliance main-body portion and the lower-jaw oral appliance main-body portion. However, the upper-jaw oral appliance main-body portion and the lower-jaw oral appliance main-body portion do not necessarily need to include the dentition impressions as long as the upper-jaw oral appliance main-body portion and the lower-jaw oral appliance main-body portion each have a shape in which the dentition fit, and may include the dentition impression portions in only a part of the upper-jaw oral appliance main-body portion and the lower-jaw oral appliance main-body portion.

The upper-jaw oral appliance and the lower-jaw oral appliance may be formed by a plurality of materials, and may be made by combining a portion formed by an organic matter and a portion formed by an inorganic matter.

The abutting surfaces of the upper-jaw oral appliance and the lower-jaw oral appliance may be substantially planar. When the abutting surfaces are substantially planar, the impact caused by the abutment of the upper-jaw oral appliance and the lower-jaw oral appliance when the jaw of the user wearing the oral appliance moves to the left and the right or in the mouth-opening direction is dispersed to the entire abutting surfaces, and hence the teeth, the jaw, and the like of the user can be protected from the impact described above.

In Embodiment 4 and Embodiment 5, the fixed member and the sliding member are integrally formed with the attachment portions thereof. In Embodiment 6 to Embodiment 8, the fixed member and the sliding member have groove portions into which the attachment portions are fitted. However, the fixed member and the sliding member may have groove portions into which the attachment portions are fitted in Embodiment 4 and Embodiment 5, and the fixed member and the sliding member may be integrally formed with the attachment portions thereof in Embodiment 6 to Embodiment 8. The shape of the attachment portion is not limited. The attachment portion may be a flat-plate-like member extending in the left-right direction in Embodiment 4 and Embodiment 5, and the attachment portion may be formed in a curved shape along the shape of the front-side surface of the upper-jaw oral appliance or the lower-jaw oral appliance in Embodiment 6 to Embodiment 8.

### [Effects of Embodiment 4 to Embodiment 8]

According to Embodiment 4 to Embodiment 8, the stopper limits the rotation of the male screw with respect to the axial direction, and hence unintentional positional misalignment of the upper-jaw oral appliance and the lower-jaw oral appliance caused when the screwing amount between the male screw and the female screw changes while the oral appliance is being worn, for example, hardly occurs.

According to Embodiment 4 to Embodiment 8, the rotation of the male screw can be regulated by the stopper that is a relatively small member, and hence the positional misalignment of the upper-jaw oral appliance and lower-jaw oral appliance can be suppressed without upsizing the oral appliance, and the discomfort of the user is hardly increased.

According to Embodiment 4 to Embodiment 8, the lower-jaw advancing apparatus is disposed on the front side of the oral appliance, and the engagement portion of the male screw into which a screwdriver can be inserted is disposed on the frontmost side in the oral appliance. Therefore, in the oral appliance according to Embodiment 4 to Embodiment 8, the advance amount of the lower-jaw oral appliance with respect to the upper-jaw oral appliance can be adjusted while the oral appliance is being worn without removing the oral appliance, and the adjustment of the advance amount is easier.

### [Configuration Example of Oral Appliance according to Second Aspect]

According to the second aspect of the present invention, there is provided an oral appliance including: an upper-jaw oral appliance and a lower-jaw oral appliance; a female screw; a male screw; a fixed member that is fixed to the upper-jaw oral appliance or the lower-jaw oral appliance and retains the female screw; and a sliding member that is fixed to the lower-jaw oral appliance or the upper-jaw oral appliance and retains the male screw. In the oral appliance, the male screw and the female screw serve to position the sliding member with respect to the fixed member in the front-rear direction by the rotation of the male screw threaded into the female screw, and position the lower-jaw oral appliance with respect to the upper-jaw oral appliance in the front-rear direction, and the oral appliance further includes a stopper that regulates rotation of the male screw with respect to the female screw with respect to the axial direction.

The oral appliance can be small-sized and can suppress the positional misalignment of the upper-jaw oral appliance and the lower-jaw oral appliance.

According to the second aspect of the present invention, the oral appliance described above in which the stopper intermittently regulates the rotation of the male screw in accordance with the rotation of the male screw is provided.

The oral appliance enables the adjuster that rotates the male screw to perceive the rotation of the male screw amount in accordance with the number of times of the collision noise or the vibration described above. Therefore, the adjuster that readjusts the positions of the upper-jaw oral appliance and the lower-jaw oral appliance can easily adjust the upper-jaw oral appliance and the lower-jaw oral appliance to suitable positions by causing the number of times of the collision noise or the vibration described above to match with the number of times in the previous adjustment.

According to the second aspect of the present invention, the oral appliance described above in which the male screw includes: a screw portion to be threaded into the female screw; and a rotation regulating portion including a recessed portion on an outer periphery of the rotation regulating portion, and the stopper is an elastic member that is retained by the fixed member or the sliding member and intermittently engages with the recessed portion by the rotation of the male screw is provided.

The oral appliance enables the adjuster that rotates the male screw to perceive the rotation of the male screw amount in accordance with the number of times of the collision noise or the vibration described above. Therefore, the adjuster that readjusts the positions of the upper-jaw oral appliance and the lower-jaw oral appliance can easily adjust the upper-jaw oral appliance and the lower-jaw oral appliance to suitable positions by causing the number of times of the collision noise or the vibration described above to match with the number of times in the previous adjustment.

According to the second aspect of the present invention, the oral appliance described above in which the fixed member or the sliding member includes a rotation regulating portion includes a recessed portion on an inner periphery of the rotation regulating portion, and the stopper is an elastic member that is retained by the male screw and intermittently engages with the recessed portion by the rotation of the male screw is provided.

The oral appliance enables the adjuster that rotates the male screw to perceive the rotation of the male screw amount in accordance with the number of times of the collision noise or the vibration described above. Therefore, the adjuster that readjusts the positions of the upper-jaw oral appliance and the lower-jaw oral appliance can easily adjust the upper-jaw oral appliance and the lower-jaw oral appliance to suitable positions by causing the number of times of the collision noise or the vibration described above to match with the number of times in the previous adjustment.

According to the second aspect of the present invention, the oral appliance described above in which the stopper includes: an elastic member that biases the male screw in a direction away from the female screw; and a locking member that regulates displacement of the male screw in a direction away from the female screw is provided.

The oral appliance can suppress unintentional positional misalignment of the upper-jaw oral appliance and the lower-jaw oral appliance caused when the screwing amount between the male screw and the female screw changes while the oral appliance is being worn, for example, by regulating the rotation of the male screw with respect to the axial direction by the stopper.

According to the second aspect of the present invention, the oral appliance described above in which the male screw is rotatably retained in the sliding member by the fastener that is removably attached to the sliding member is provided.

In the oral appliance, the rotation of the male screw is easy.

According to the second aspect of the present invention, the oral appliance described above in which the sliding member includes a through hole in which an end portion of the fastener can be fitted is provided.

In the oral appliance, the male screw is mountable and removable, and the maintenance is easy.

According to the second aspect of the present invention, the oral appliance described above in which the abutting surfaces of the upper-jaw oral appliance and the lower-jaw oral appliance are substantially planar is provided.

In the oral appliance, the impact caused by the abutment of the upper-jaw oral appliance and the lower-jaw oral appliance when the jaw of the user wearing the oral appliance moves to the left and the right or in the mouth-opening direction is dispersed to the entire abutting surfaces, and hence the teeth, the jaw, and the like of the user are protected from the impact described above.

This application claims priority from Japanese Patent Application No. 2017-122364 filed on June 22, 2017, Japanese Patent Application No. 2018-025034 filed on February 15, 2018, and Japanese Patent Application No. 2018-058743 filed on March 26, 2018, and the contents described in the description, the claims, and the drawings of those applications are incorporated herein by reference.

### Industrial Applicability

According to the oral appliance of the first aspect of the present invention, the displacement of the lower jaw to the rear side can be limited while adjusting the position of the lower jaw of the user wearing the oral appliance. In the oral appliance of the present invention, the lower-jaw advancing apparatus and the male screw do not protrude to the front side, and hence the wearing comfortability of the user is better than the related art. Therefore, the oral appliance of the present invention can be used for the prevention and treatment of temporomandibular joint disorder and the suppression of bruxism, for example, in addition to the prevention and treatment of sleep apnea syndrome.

According to the oral appliance of the second aspect of the present invention, the displacement of the lower jaw to the rear side can be limited while adjusting the position of the lower jaw of the user wearing the oral appliance. In the oral appliance of the present invention, the adjustment of the advance amount of the lower-jaw oral appliance is easier than the related art. Therefore, the oral appliance of the present invention can be used for the prevention and treatment of temporomandibular joint disorder and the suppression of bruxism, for example, in addition to the prevention and treatment of sleep apnea syndrome.

### Reference Signs List

1100, 1300 Oral appliance
1110 Upper-jaw oral appliance
1112 Dentition impression portion
1114 Downward facing surface
1120 Lower-jaw oral appliance
1122 Dentition impression portion
1124 Upward facing surface
1200 Lower-jaw advancing apparatus
1210, 1210a, 1210b, 1210c, 1210d Front-side retaining portion
1212, 1212b, 1212c, 1212d Firmly fixed portion
1214, 1214a, 1214b, 1214c, 1214d Front-side facing portion
1216, 1216a, 1216b, 1216c, 1216d Retaining hole
1217 Protection surface
1218 Fitting portion
1219 Protection surface
1220, 1220a, 1220b, 1220c, 1220d Rear-side retaining portion
1222, 1222a, 1222b Protruding portion
1224, 1224a, 1224b Rear side facing portion
1230 Male screw
1232 Engagement portion
1234 Front-side locking portion
1235 Cylindrical portion
1236 Rear-side locking portion
1238 Screw portion
1240, 1240d Female screw
1242 Female screw rear end portion
1252a Guide hook
1252b Hooking hook
1252c Sliding member
1254a, 1254b Guide bar
1254c Sliding member
1256a Guide rail
1256b Hammer portion
1256c Sliding member
1262a Turn retaining hole
1262b Diameter-reduced portion
1262c Stopper
1262d Turn retaining portion
1264a Through hole
1264b Insertion bar
1264c Stopper
1264d Turn retaining portion
1266a Rear-side retaining portion
1266b Rear-side retaining hole
1266c Female screw locking portion
1268a Female screw hole
1270 Pressing down portion
1400 Screwdriver
2100 Oral appliance
2110 Upper-jaw oral appliance
2112 Dentition impression portion
2120 Lower-jaw oral appliance
2122 Dentition impression portion
2200, 2300, 2300a, 2300b Lower-jaw advancing apparatus
2210, 2310 Fixed member
2212, 2312 Groove portion
2214, 2314 Cover portion
2216, 2316 Attachment portion
2216a, 2316a Through hole
2220, 2320 Sliding member
2222, 2322 Groove portion
2222a Recessed portion
2224, 2324 Cover portion
2224a, 2224b Through hole
2224c Plate portion
2226, 2326 Attachment portion
2226a, 2326a Through hole
2228 Fastener
2228a, 2228b Fitting portion
2228c Curved portion
2230, 2330 Male screw
2232, 2332 Engagement portion
2232a Recessed portion
2234, 2236, 2334 Collar portion
2235 Narrow portion
2238, 2338, 2338a Screw portion
2240, 2340 Female screw
2250, 2350, 2350a Stopper
2252 End portion
2254 First plate-like portion
2255 Fold-over portion
2256 Second plate-like portion
2258 Claw portion
2260 Rotation regulating portion
2270 Stopper
2272 End portion
2274 Plate-like portion
2278 Claw portion
2280 Rotation regulating portion
2314a Groove portion
2324a Groove portion
2328 Fastener
2328a Insertion through hole
2335 Cylindrical portion
2336, 2336a, 2336b Narrow portion
2338b Elastic coating
2352 Compression coil spring
2354, 2354a, 2354b Locking member
2355 Locking piece
2356 Protruding locking portion
2400 Screwdriver

## Claims

1. An oral appliance, comprising:
one pair of an upper-jaw oral appliance and a lower-jaw oral appliance facing each other;
a male screw;
a female screw;
a front-side retaining portion that is disposed on one oral appliance out of the upper-jaw oral appliance and the lower-jaw oral appliance and retains the male screw or the female screw; and
a rear-side retaining portion that is disposed on another oral appliance out of the upper-jaw oral appliance and the lower-jaw oral appliance and retains the female screw or the male screw behind the front-side retaining portion, wherein
the female screw and the male screw serve to position the lower-jaw oral appliance with respect to the upper-jaw oral appliance in a front-rear direction by rotation of the male screw threaded into the female screw.

2. The oral appliance according to claim 1, wherein the front-side disposing portion is disposed on a dentition surface of the one oral appliance or behind the dentition surface.

3. The oral appliance according to claim 1 or 2, wherein the rear-side retaining portion comprises:
a protruding portion protruding to a tongue portion side; and
a rear-side facing portion that is disposed behind a dentition by the protruding portion, and retains the female screw or the male screw by facing the front-side retaining portion.

4. The oral appliance according to any one of claims 1 to 3, wherein:
the front-side retaining portion retains the male screw or the female screw such that the male screw or the female screw is displaceable or turnable in a left-right direction; and
the rear-side retaining portion retains the female screw or the male screw such that the female screw or the male screw is displaceable or turnable in the left-right direction.

5. The oral appliance according to claim 4, wherein:
the rear-side retaining portion comprises a turn member that turns in the left-right direction;
the female screw or the male screw is retained by the rear-side retaining portion such that the female screw or the male screw is fixed to the turn member and is turnable in the left-right direction; and
the male screw is allowed to turn in the left-right direction.

6. The oral appliance according to claim 5, wherein the turn member comprises:
a turn retaining portion that retains the female screw or the male screw; and
a stopper that fixes a position of the turn retaining portion such that the turn retaining portion is turnable.

7. The oral appliance according to any one of claims 4 to 6, wherein:
the front-side retaining portion comprises a retaining hole formed so as to have a wide width in the left-right direction;
the male screw or the female screw is retained by the front-side retaining portion so as to be displaceable in the left-right direction by being inserted through the retaining hole; and
the male screw is allowed to be displaced or turn in the left-right direction.

8. The oral appliance according to any one of claims 4 to 6, wherein:
the front-side retaining portion comprises a sliding member that is displaced in the left-right direction;
a position of the male screw or the female screw is fixed with respect to the sliding member;
the male screw or the female screw is retained by the front-side retaining portion; and
the male screw is allowed to be displaced or turn in the left-right direction.

9. The oral appliance according to claim 4, wherein:
the front-side retaining portion comprises an opening portion formed so as to have a wide width in the left-right direction;
the male screw or the female screw is retained by the front-side retaining portion by being inserted in the opening portion;
the rear-side retaining portion comprises an opening portion formed so as to have a wide width in the left-right direction;
the female screw or the male screw is retained by the rear-side retaining portion by being inserted in the opening portion; and
the male screw is allowed to be displaced in the left-right direction.

10. The oral appliance according to any one of claims 1 to 9, wherein the rear-side retaining portion comprises a pressing down portion that presses down a tongue portion of a user when a mouth is closed.

11. The oral appliance according to any one of claims 1 to 10, further comprising a stopper that regulates rotation of the male screw with respect to the female screw with respect to an axial direction.

12. The oral appliance according to claim 11, wherein the stopper intermittently regulates the rotation of the male screw in accordance with the rotation of the male screw.

13. The oral appliance according to claim 12, wherein:
the male screw comprises:
a screw portion to be threaded into the female screw; and
a rotation regulating portion comprising a recessed portion on an outer periphery of the rotation regulating portion; and
the stopper is an elastic member that is retained by the front-side retaining portion or the rear-side retaining portion and intermittently engages with the recessed portion by the rotation of the male screw.

14. The oral appliance according to claim 12, wherein:
the front-side retaining portion or the rear-side retaining portion comprises a rotation regulating portion comprising a recessed portion on an inner periphery of the rotation regulating portion; and
the stopper is an elastic member that is retained by the male screw and intermittently engages with the recessed portion by the rotation of the male screw.

15. The oral appliance according to claim 11, wherein the stopper comprises:
an elastic member that biases the male screw in a direction away from the female screw; and
a locking member that regulates displacement of the male screw in the direction away from the female screw.

16. The oral appliance according to claim 11, wherein the stopper comprises:
a locking piece formed so as to protrude from the male screw;
a protruding locking portion that is formed so as to protrude from an inner periphery of the front-side retaining portion or the rear-side retaining portion, and regulates the rotation of the male screw by being engaged with the locking piece; and
a contact switching portion that switches between a state in which the locking piece and the protruding locking portion are engaged with each other and the rotation of the male screw is regulated, and a state in which the locking piece and the protruding locking portion are not engaged with each other and the rotation of the male screw is not regulated.
